# EUROPEAN PATENT APPLICATION

(11) **EP 4 159 720 A1**
(43) Date of publication of application: **05.04.2023**
(21) Application number: 22195637.8
(22) Date of filing: 14.09.2022
(51) Int. Cl.: C07D 213/81, A61P 31/00, A61P 35/00, A61K 31/4412

(54) **NONSYMMETRIC SUBSTITUTED 1,1'-BIPHENYL DERIVATIVES AND USES THEREOF**

(30) Priority: 30.09.2021 PL 43910721; 09.08.2022 PL 44198222
(71) Applicant: Recepton Spolka z Ograniczona Odpowiedzialnoscia, 80-172 Gdansk (PL)
(72) Inventor: Hec, Aleksandra, 32-065 Krzeszowice (PL); Barczynski, Jan, 80-723 Gdansk (PL); Blaszkiewicz, Urszula, 31-216 Krakow (PL); Bielski, Przemyslaw, 06-458 Niedzborz (PL); Mikitiuk, Michal, 80-126 Gdansk (PL); Sitar, Tomasz, 80-176 Gdansk (PL); Holak, Tadeusz, 30-150 Krakow (PL)
(74) Representative: Dominiak, Malgorzata Maria

(57) **Abstract**

The subject of the present invention are new nonsymmetrically substituted 1,1'-biphenyl derivatives of formula (I) as small-molecule inhibitors targeting the PD-1/PD-L1 immune checkpoint that may find application in cancer immunotherapy.

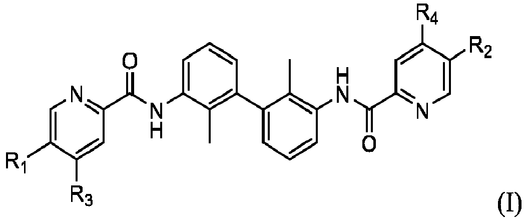

## Description

### FIELD OF INVENTION

The subject of the invention is novel nonsymmetric 1, 1'-biphenyl derivative of general formula (I) or a pharmaceutically acceptable salt or a stereoisomer thereof as small molecule inhibitor targeting the PD-1/PD-L1 immune checkpoint that can be used in medicine, preferably in treatment and/or prophylaxis of malignant neoplastic diseases or disorders or in stimulating and/or increasing an immune response in a patient in response to an infectious disease. The disclosure provides compounds and methods of their use.

### BACKGROUND OF THE INVENTION

Immunotherapy based on inhibitors of checkpoints, such as PD-1, CTLA-4, etc. results in sustained therapeutic responses and better survival in patients with various types of cancer [1-3]. Advances in this area led the Science magazine to declare immunotherapy "Breakthrough of the Year" in 2013, while in 2018 James P. Allison from the US and Tasuku Honjo from Japan received the Nobel Prize in Medicine and Physiology for developing methods to unlock the immune system.

One of the ways in which cancer cells evade immune surveillance by allowing tumors to grow is through complex immunosuppressive mechanisms, which usually play a role in controlling normal antibacterial and antiviral immune responses and prevent excessive immune responses and associated immunopathologies.

For example, strongly or chronically activated T cells upregulate PD-1 expression, resulting in cells that express PD-L1 upon exposure to T cell-derived IFNγ. Thus, in a process known as "adaptive immune resistance", the anti-tumor T cell response will suppress activated CD8 + T cells via PD-L1/PD-1.

PD-1, or programmed cell death receptor 1 (CD279), hereafter named as PD-1, PD-1 protein or PD-1 receptor, is a 55 kDa glycoprotein protein consisting of 288 amino acids from the B7-CD28 protein family. It consists of an extracellular IgV-like domain, a transmembrane and a cytoplasmic domain. PD-1 acts as a negative regulator of the immune response. It is encoded by the pdcd1 gene located on the second chromosome (2q37) and is expressed on the surface of activated T and B lymphocytes, responsible for the regulation of immune tolerance.

The PD-1 ligand (PD-L1, CD270 or B7-H1), hereafter also named as PD-L1 protein/protein receptor, is a 33 kDa glycoprotein protein with the gene located on chromosome 9p24. This type I transmembrane protein consists of 290 amino acids and has IgV and IgC domains in the extracellular part. Its overexpression has been observed in many cells of the immune system, such as macrophages, B lymphocytes, and many types of non-hematopoietic cells [4]. The interaction between PD-1 and its PD-L1 ligand results in the suppression of T cell proliferation, cytokine secretion and lymphocyte cytotoxic functions. This pathway is also often used by cancer cells as well as viruses and bacteria to weaken the body's natural immune response and facilitate its spread throughout the attacked organism [5]. Blockade of this pathway, e.g. by specific antibodies blocking the action of PD1-/PD-L1, restores cytotoxic functions of T lymphocytes, normalizing the immune response of the organism and is one of the most important elements of cancer immunotherapy.

At present, all registered or undergoing advanced clinical trials compounds that directly block the interaction of the PD-1 protein with its ligand are based on the monoclonal antibodies or their combinations with each other or with chemotherapy. However, small-molecule inhibitors of the PD-1/PD-L1 interaction are an interesting alternative to antibody-based therapies due to the much lower production costs, the possibility of oral instead of intravenous administration, better pharmacokinetics and potentially no adverse effects related to the patient's immunity so characteristic of therapies based on antibodies [6].

WO2019/149183A1 and WO2021/008491A1 describe some biphenyl derivatives useful as inhibitors of the PD-1/PD-L1 interaction, but there are the compounds of a fully symmetrical structure with varying degrees of biological activity as determined by the NFAT assay.

The aim of the invention is to provide new compounds that could be used as small-molecule inhibitors of the PD-1/PD-L1 interaction.

### SUMMARY

The present disclosure provides a compound of general formula (I): 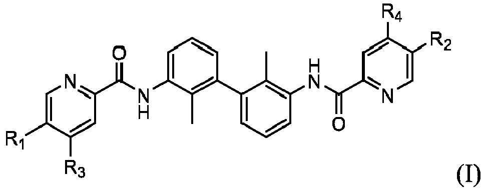 or a pharmaceutically acceptable salt or a stereoisomer thereof.

Compounds of the invention may be used as nonsymmetric inhibitors of the PD-1/PD-L1 interaction that bind to, dimerize PD-L1 and modulate the PD-1/PD-L1 interaction.

The inventors of the present invention developed a series of compounds of general formula (I) that have a nonsymmetrical structure and are selected based on the proprietary modelling method based on the X-ray cocrystals structures of human PD-L1 complexed with small molecular weight inhibitors (PDB codes 5J89, 5J8O and Zak, K.M.; Grudnik, P.; Magiera, K.; Domling, A.; Dubin, G.; Holak,T .A. Structural Biology of the Immune Checkpoint Receptor PD-land Its Ligands PD-L1/PD-L2. Structure 2017, 25, 1163-1174). These X-ray structures and NMR studies indicate that one small-molecule inhibitor that binds to PD-L1 is located at the interface of the asymmetric PD-L1 dimer (Guzik, K.; Zak, K.M.; Grudnik, P.; et al. Small-Molecule Inhibitors of the Programmed Cell Death-1/Programmed Death-Ligand 1 (PD-1/PD-L1) Interaction via Transiently Induced Protein States and Dimerization of PD-L1. J. Med. Chem. 2017, 60, 5857-5867; Skalniak, L.; Zak, K. M.; Guzik, K. et al. Small-Molecule Inhibitors of PD-1/PD-L1 Immune Checkpoint Alleviate the PD-L1-Induced Exhaustion of T-Cells. Oncotarget 2017, 8, 72167-72181). It is therefore believed that the *nonsymmetric* PD-1/PD-L1 inhibitors should be more effective at forming of asymmetric PD-L1 dimers than the corresponding compounds that have the same groups on both sides of the biphenyl unit. This approach produced compounds with a cut-off of EC₅₀ (half-maximal effective concentration) in the NFAT cellular assay below 500 nM (Table 1). The PD-1/PD-L1 inhibitors that exhibit EC₅₀ higher than this cut-off are considered not potent enough to stimulate T-cells to clinically relevant anti-cancer activity.

The compounds of the invention can be used in a pure form or as a pharmaceutically acceptable salt or a stereoisomer thereof which find use as a medicament, preferably in the treatment of neoplastic diseases. These inhibitors bind to PD-L1, dimerize the PD-L1 protein, preventing its interaction with the PD-1 protein, which was confirmed by NMR using the methodology described by Zak et al. 2015 (Zak, K.M.; Grudnik, P.; Guzik, K.; Zieba, B.J.; Musielak, B.; Dömling, A.; Dubin, G.; Holak, T.A. Structural Basis for Small Molecule Targeting of the Programmed Death Ligand1 (PD-L1). Oncotarget 2016, 7, 30323-30335), Guzik et al. 2017 (Guzik, K.; Zak, K.M.; Grudnik, P.; et al. Small-Molecule Inhibitors of the Programmed Cell Death-1/Programmed Death-Ligand 1 (PD-1/PD-L1) Interaction via Transiently Induced Protein States and Dimerization of PD-L1. J. Med. Chem. 2017, 60, 5857-5867), and Skalniak et al., 2017 (Skalniak, L.; Zak, K. M.; Guzik, K. et al. Small-Molecule Inhibitors of PD-1/PD-L1 Immune Checkpoint Alleviate the PD-L1-Induced Exhaustion of T-Cells. Oncotarget 2017, 8,72167-72181).

The present disclosure further provides a compound of the invention, or a pharmaceutically acceptable salt or a stereoisomer thereof, for use as a medicament, especially in treatment and/or prophylaxis of malignant neoplastic diseases or disorders or in stimulating and/or increasing an immune response in a patient in response to an infectious disease, wherein the patient is a mammal, preferably a human.

Additionally, compound of general formula (I) or a salt or stereoisomer thereof can be used for inhibiting growth of tumor cells in vitro.

### DETAILED DESCRIPTION

The compounds of the invention are compounds of general formula (I): 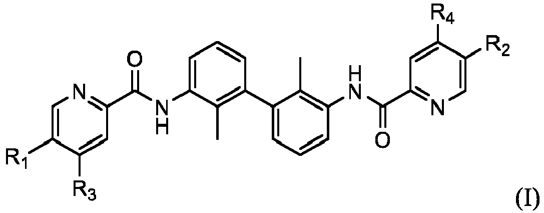 or a pharmaceutically acceptable salt or a stereoisomer thereof, wherein:
R₁ and R₂ are each independently selected from the group consisting of 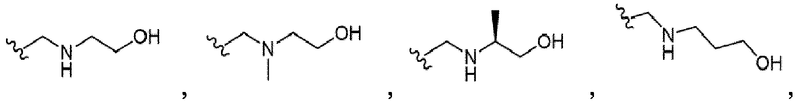 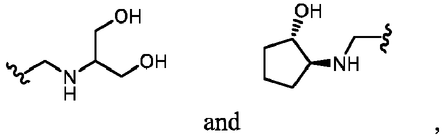 wherein R₁ is different from R₂,
R₃ and R₄ are each independently selected from -H or -OCH₃,
and wherein at least one of R₃ and R₄ is the -OCH₃ group.

In some embodiments, the present disclosure provides compounds having Formula (I) or pharmaceutically acceptable salt or stereoisomer thereof, wherein the substituent R₃ is the - OCH₃ group.

In some embodiments, the present disclosure provides compounds having Formula (I) or pharmaceutically acceptable salt or stereoisomer thereof, wherein the substituent R₄ is the - OCH₃ group.

Preferably, the compound of general formula (I) is selected from:
5-((((1S,2S)-2-hydroxycyclopentyl)amino)methyl)-N-(3'-(5-((((S)-1-hydroxypropan-2-yl)amino)methyl)-4-methoxypicolinamido)-2,2' -dimethyl-[1,1'-biphenyl]-3-yl)-4 - methoxypicolinamide;
(S)-5-(((2-hydroxyethyl)(methyl)amino)methyl)-N-(3'-(5-(((1-hydroxypropan-2-yl)amino)methyl)-4-methoxypicolinamido)-2,2'-dimethyl-[1,1'-biphenyl]-3-yl)-4 - methoxypicolinamide;
5-(((1,3-dihydroxypropan-2-yl)amino)methyl)-N-(3'-(5-((((1R,2R)-2-hydroxycyclopentyl)amino)methyl)-4-methoxypicolinamido)-2,2'-dimethyl-[1,1'-biphenyl]-3-yl)-4-methoxypicolinamide;
(S)-5-(((1,3-dihydroxypropan-2-yl)amino)methyl)-N-(3'-(5-(((1-hydroxypropan-2-yl)amino)methyl)-4-methoxypicolinamido)-2,2'-dimethyl-[1,1'-biphenyl]-3 -yl)-4-methoxypicolinamide;
5-(((2-hydroxyethyl)(methyl)amino)methyl)-N-(3'-(5-(((2-hydroxyethyl)(methyl)amino)methyl)picolinamido)-2,2'-dimethyl-[1,1'-biphenyl]-3-yl)-4-methoxypicolinamide;
5-((((1R,2R)-2-hydroxycyclopentyl)amino)methyl)-N-(3'-(5-(((2-hydroxy ethyl) (methyl)amino)methyl)picolinamido)-2,2'-dimethyl-[1,1'-biphenyl]-3-yl)-4-methoxypicolinamide;
5-((((1R,2R)-2-hydroxycyclopentyl)amino)methyl)-N-(3'-(5-(((2-hydroxyethyl)(methyl)amino)methyl)picolinamido)-2,2'-dimethyl-[1,1'-biphenyl]-3-yl)-4-methoxypicolinamide trihydrochloride;
(S)-5-(((1-hydroxypropan-2-yl)amino)methyl)-N-(3 '-(5-(((3-hydroxypropyl)amino)methyl)-4-methoxypicolinamido)-2,2'-dimethyl-[1,1'-biphenyl]-3-yl)-4-methoxypicolinamide;
5-(((2-hydroxyethyl)amino)methyl)-N-(3'-(5-(((3-hydroxypropyl)amino)methyl)-4-methoxypicolinamido)-2,2'-dimethyl-[1,1'-biphenyl]-3-yl)-4-methoxypicolinamide;
(S)-N-(3'-(5-(((2-hydroxyethyl)(methyl)amino)methyl)picolinamido)-2,2'-dimethyl-[1,1'-biphenyl]-3-yl)-5-(((1-hydroxypropan-2-yl)amino)methyl)-4-methoxypicolinamide;
or a pharmaceutically acceptable salt or a stereoisomer thereof.

In another aspect the invention concerns the compound of general formula (I) or a pharmaceutically acceptable salt or a stereoisomer thereof for use as a medicament, preferably in treatment and/or prophylaxis of malignant neoplastic diseases or disorders or in stimulating and/or increasing an immune response in a patient in response to an infectious disease, wherein the patient is a mammal, preferably a human.

Preferably, the disease or disorder spreads through an organism through a PD-1/PD-L1 signaling pathway and the compound of general formula (I) or a pharmaceutically acceptable salt or a stereoisomer thereof is used as a modulator of the PD-1/PD-L1 signaling pathway.

Preferably, the compound of general formula (I) or a pharmaceutically acceptable salt or a stereoisomer thereof binds to PD-L1 protein receptor and/or dimerizes PD-L1 protein receptor and/or inhibits PD-1/PD-L1 interaction.

Compounds of the present disclosure can inhibit the PD-1/PD-L1 protein-protein interaction and, thus, are useful in treating diseases and disorders associated with activity of PD-1 and the diseases and disorders associated with PD-L1 including its interaction with other proteins such as PD-1 and B7-1 (CD80).

The compounds of the present invention can be used for inhibiting growth of tumor cells *in vitro* by contacting a tumor cell or tissue *in vitro* with a compound of formula (I) or with a salt or stereoisomer thereof.

The compounds of the present disclosure, or pharmaceutically acceptable salts or stereoisomers thereof, are useful for therapeutic administration to enhance, stimulate and/or increase immunity in cancer. The compounds of the present disclosure can be used alone, in combination with other agents or therapies or as an adjuvant or neoadjuvant for the treatment of cancer.

The compounds of the present disclosure inhibit the PD-1/PD-L1 protein-protein interaction, resulting in a PD-1 pathway blockade. The blockade of PD-1/PD-L1 can enhance the immune response to cancerous cells and infectious diseases in mammals, including humans.

The compounds of general formula (I) can be used for inhibiting growth of tumor cells in an individual or a patient. Examples of cancers include those whose growth may be inhibited using compounds of the disclosure and cancers typically responsive to immunotherapy.

The compounds of the present disclosure can be used for the treatment of metastatic cancers, especially metastatic cancers that express PD-L1.

Cancers treatable with compounds of general formula (I) or their combinations include melanoma (e.g., metastatic malignant melanoma), renal cancer (e.g., clear cell carcinoma), prostate cancer (e.g., hormone refractory prostate adenocarcinoma), breast cancer, colon cancer and lung cancer (e.g., non-small cell lung cancer). Additionally, the disclosure includes refractory or recurrent malignancies whose growth may be inhibited using the compounds of the disclosure.

The present invention is further explained in detailed below with reference to examples, which are not intended to limit scope the present invention.

### Description of figures:

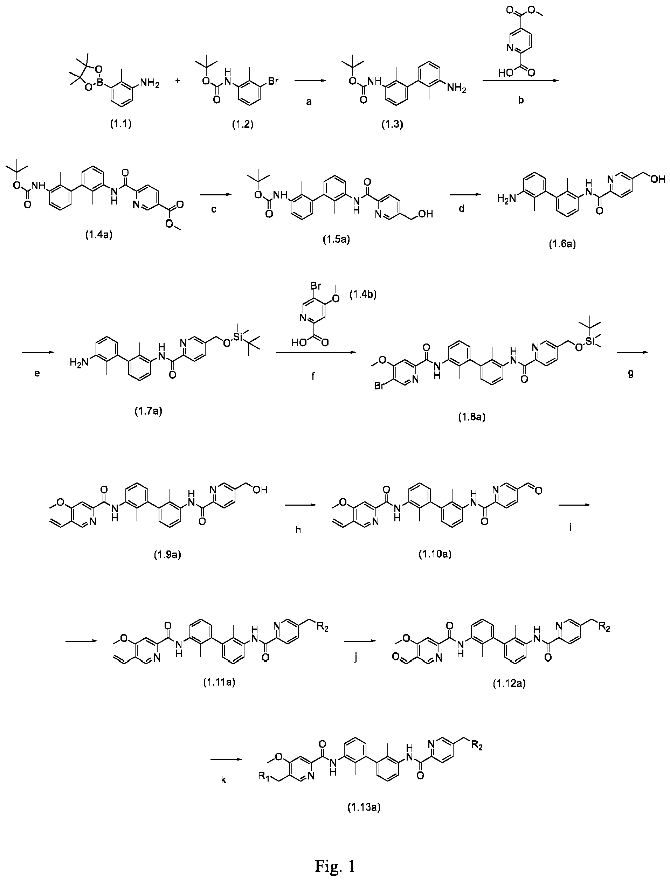

Fig. 1 presents a general scheme for synthesis of unsymmetrical 1,1'-biphenyl derivatives, where R₁ ≠ R₂, R₃ = -OMe and R₄ = -H and a) Pd(dppf)Cl₂ ^{∗} DCM, K₂CO₃, 1,4-dioxane/H₂O, 90°C; b) HATU, DIPEA, THF; c) LiBH₄, THF, MeOH; d) 6M HCl in IPA, DCM; e) TBDMSCI, Imidazole, DCM, THF; f) HATU, DIPEA, THF; g) potassium trifluoro(vinyl)borate, Pd(dppf)Cl₂, K₂CO₃, 90°C; h) Dess-Martin reagent, DCM; i) amine, reducing agent - reductive amination (protection of reactive groups in the amine, e.g. the free group -OH); j) K₂OsO₄, NaIO₄, 1,4-dioxane/H₂O; k) amine, reducing agent - reductive amination (deprotection of all groups e.g. TBDMS-p. TBDMS-) or reduction (LiBH₄), chloride formation (MsCl, TEA, 0°C), aliphatic halide substitution (amine, KI, K₂CO₃, ACN, 50°C).

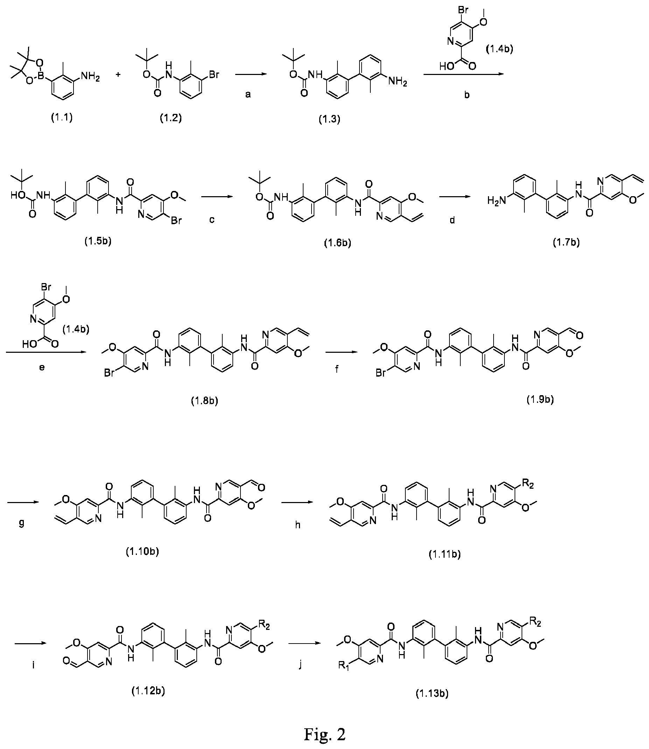

Fig. 2 presents a general scheme for synthesis of unsymmetrical 1, 1'-biphenyl derivatives, where R₁ + R₂, R₃ = R₄ = -OMe and a) Pd(dppf)Cl₂ ^{∗} DCM, K₂CO₃, 1,4-dioxane/H₂O, 90°C; b) EDC HCl, HOBt, DIPEA, DMF; c) potassium trifluoro (vinyl) borate, Pd(dppf)Cl₂, K₂CO₃, 90°C; d) 6M HCl in IPA, DCM; e) HATU, DIPEA, THF; f) K₂OsO₄, NaIO₄, 1,4-dioxane/H₂O; g) potassium trifluoro (vinyl) borate, Pd(dppf)Cl₂, K₂CO₃, 90°C; h) amine, reducing agent - reductive amination (protection of reactive groups in the amine, e.g. the free group -OH); i) K₂OsO₄, NaIO₄, 1,4-dioxane/H₂O; j) amine, reducing agent - reductive amination (deprotection of all groups e.g. TBDMS-) or reduction (LiBH4), chloride formation (MsCl, TEA, 0°C), aliphatic halide substitution (amine, KI, K₂CO₃, ACN, 50°C).

### Embodiments of the invention:

The compound structure of the present invention is determined by nuclear magnetic resonance (NMR) or liquid chromatography-mass spectrometry (LC/MS). UHPLC-MS/MS analysis was performed on a Waters ACQUITY UPLC instrument (Waters Corporation, Milford, MA, USA) coupled to a Waters TQD mass spectrometer. DAD chromatograms were recorded using a Waters eλ PDA detector. ¹H and ¹³CNMR spectra were recorded on a Bruker Avance 600 MHz spectrometer expressing chemical shifts (δ) in ppm and coupling constants (J) in Hz. TMS was used as a standard and an internal standard. In addition, chemical shifts were analyzed with respect to the solvent peaks (DMSO-d₆, CDCl₃). The compounds were purified by column chromatography using an Interchim-Flash-puriFlash XS-520Plus flash chromatograph. The progress of the reaction was monitored by visualization of Merck 60 254 to 365 nm silica gel TLC plates.

The compounds of Formula (I) can be prepared according to the synthetic methods set out in the description below. It should be noted that the absolute configuration of the chiral center is illustrative only, and these schemes may be used to synthesize any stereoisomers (or any mixtures thereof).

### Synthesis of compounds, where R₃ = -OMe, R₄ = -H:

### Example 1. Preparation of 5-((((1S,2S)-2-hydroxycyclopentyl)amino)methyl)-N-(3'-(5-(((2-hydroxyethyl)(methyl)amino)methyl)picolinamido)-2,2'-dimethyl-[1,1'-biphenyl]-3-yl)-4-methoxypicolinamide - Compound (R1_C451)

### Preparation of Intermediates:

### Intermediate (1.1): Synthesis of 2-methyl-3-(4,4,5,5-tetramethyl-1,3,2-dioxaborolan-2-yl)aniline

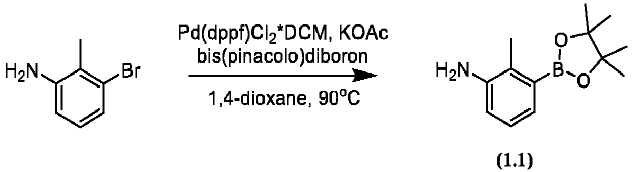

A suspension of 3-bromo-2-methylaniline (2 g, 10.75 mmol, 1 eq), bis(pinacol) diboron (4.095 g, 16.12 mmol, 1.5 eq) and potassium acetate (3.165 g, 32.25 mmol, 3 eq) in 1,4-Dioxane (40 ml, 20 eq) was purged with argon for 15 min. Then Pd(dppf)Cl₂^{∗}DCM (0.878 g, 1.07 mmol, 0.1 eq) was added and allowed to stir at 90°C under argon for 4 days. After cooling to room temperature, the mixture was diluted with EtOAc and extracted. The organic layers were combined, dried over Na₂SO₄, filtered, and concentrated under reduced pressure. The crude product was purified by flash chromatography using Hex: EtOAc in gradient (0-100%) to afford compound (1.1) (3.32 g) as a yellowish solid.

¹H NMR (600 MHz, DMSO) δ 6.87 - 6.83 (m, 2H), 6.72 - 6.69 (m, 1H), 4.72 (s, 2H), 2.20 (s, 3H), 1.28 (s, 12H).

### Intermediate (1.2): Synthesis of tert-butyl (3-bromo-2-methylphenyl)carbamate

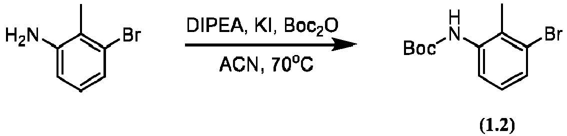

3-Bromo-2-methylaniline (8 g, 43 mmol, 1 eq) was dissolved in ACN (160 mL, 20 eq). Then DIPEA (22.469 ml, 129 mmol, 3 eq) and di-tert-butyl dicarbonate (12.2 g, 55.9 mmol, 1.3 eq) and KI (7.138 g, 43 mmol, 1 eq) were added and allowed to stir at 70°C for 2 days. The reaction was then diluted with DCM and the resulting organic solution was washed with saturated aqueous NaHCO₃ followed by brine. The organic layer was dried under sodium sulphate, concentrated and the crude mixture was purified by silica gel column chromatography eluting with a gradient of 15% of EtOAc in hexane to afford compound **(1.2)** (10 g, 81% yield) as a white solid.

¹H NMR (300 MHz, DMSO) δ 8.80 (s, 1H), 7.43 - 7.36 (m, 1H), 7.32 - 7.22 (m, 1H), 7.13 - 7.04 (m, 1H), 2.24 (d, J = 2.7 Hz, 3H), 1.45 (d, J = 2.8 Hz, 9H).

### Intermediate (1.3): Synthesis of tert-butyl (3'-amino-2,2'-dimethyl-[1,1'-biphenyl]-3-yl)carbamate

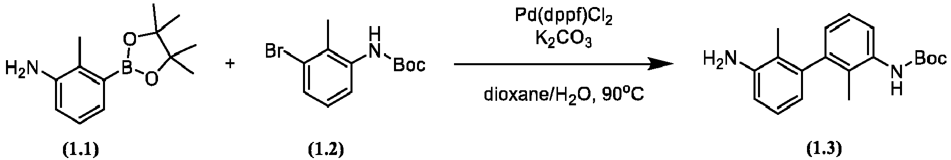

Compound **(1.1)** (2.75 g, 11.8 mmol, 1 eq), **(1.2)** (3.376 g, 11.80 mmol, 1 eq), K₂CO₃ (4.891 g, 35.39 mmol, 3 eq) was added to the flask and dissolved in a mixture of water (27.5 mL, 10 eq) and 1,4-dioxane (50 mL, 20 eq.). The solution was degassed by argon purging for 15 min. Then a palladium catalyst (0.863 g, 1.18 mmol, 0.1 eq) was added and the reaction was allowed to stir at 90°C overnight. After this time, water was added and the reaction was extracted with EtOAc. The organic layer was washed with brine, dried over Na₂SO₄ and evaporated. The crude product was purified by flash chromatography using a Hex: EtOAc in gradient (0-100%) to afford compound (1.3) (3.48 g, 94% yield) as yellowish oil.

¹H NMR (300 MHz, DMSO) δ 8.51 (s, 1H), 7.26 (d, J = 8.0 Hz, 1H), 7.14 (t, J = 7.8 Hz, 1H), 6.92 (d, J = 7.7 Hz, 1H), 6.83 (dd, J = 7.5, 1.5 Hz, 1H), 6.62 (dd, J = 8.0, 1.3 Hz, 1H), 6.25 (dd, J = 7.4, 1.3 Hz, 1H), 4.86 (s, 2H), 1.85 (d, J = 3.3 Hz, 3H), 1.70 (s, 2H), 1.45 (d, J = 3.1 Hz, 9H).

LC/MS: RT= 6.20 min, purity: 97%, [M+H⁺]= 313.31

### Intermediate (1.4a): Synthesis of methyl 6-((3'-((tert-butoxycarbonyl)amino)-2,2'-dimethyl-[1,1'-biphenyl]-3-yl)carbamoyl)nicotinate

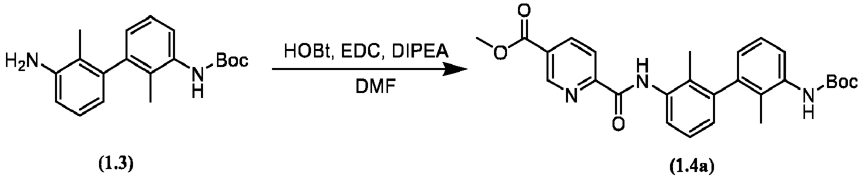

To a solution of 5- (methoxycarbonyl) picolinic acid (2.319 g, 12.80 mmol, 1 eq) in DMF (40 ml, 10 eq) was added DIPEA (6.69 ml, 25.61 mmol, 2 eq.). The mixture was cooled to 0°C and treated with EDC-HCl (4.909 g, 25.61 mmol, 2 eq), HOBt (3.922 g, 25.61 mmol, 2 eq) and **(1.3)** (4 g, 12.8 mmol, 1 eq). The reaction was stirred at room temperature overnight. Water was then added, and the reaction was extracted with DCM. The organic layer was washed with brine, dried over Na2SO4 and evaporated. The crude product was purified by flash chromatography using DCM: MeOH (0-8%) to afford compound **(1.4a)** (5.33 g, 88% yield) as yellowish solid.

¹H NMR (300 MHz, DMSO) δ 10.46 (s, 1H), 9.19 (dt, J = 3.4, 1.7 Hz, 1H), 8.61 - 8.52 (m, 2H), 8.35 - 8.28 (m, 1H), 7.74 (d, J = 8.0 Hz, 1H), 7.33 (t, J = 6.2 Hz, 2H), 7.21 (t, J = 7.7 Hz, 1H), 7.01 - 6.90 (m, 2H), 3.95 (d, J = 1.9 Hz, 3H), 1.97 (d, J = 3.0 Hz, 3H), 1.90 (s, 3H), 1.49 - 1.43 (m, 9H).

### Intermediate (1.5a): Synthesis of tert-butyl (3'-(5-(hydroxymethyl)picolinamido)-2,2'-dimethyl-[1,1'-biphenyl]-3-yl)carbamate

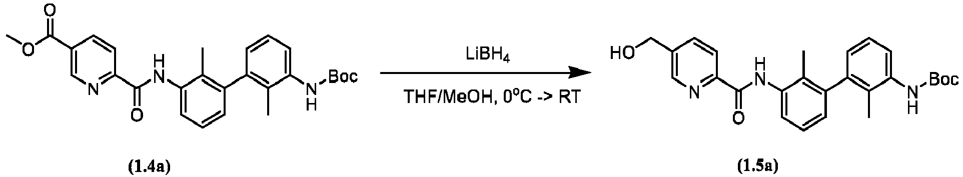

Compound **(1.4a)** (5.33 g, 11.1 mmol. 1 eq) was dissolved in a mixture of THF/MeOH (2:1) (79.75 ml) and cooled to 0°C with an ice bath. A solution of 4M LiBH₄ (8.406 ml, 33.62 mmol, 3 eq) was then added dropwise and the reaction mixture was stirred at this temperature for 15 min and then allowed to warm to RT. The reaction was monitored on a TLC plate. After the starting material was completely consumed (15 min), water was added and the solvent was evaporated. DCM was added to the residue and the reaction mixture was extracted with DCM/water. The organic layer was washed with brine, dried over Na₂SO₄ and evaporated to afford **(1.5a)** (4.46 g, 89% yield) as yellowish solid.

¹H NMR (300 MHz, DMSO) δ 10.32 (s, 1H), 8.66 (d, J = 2.3 Hz, 1H), 8.59 (s, 1H), 8.18 - 8.12 (m, 1H), 8.03 - 7.97 (m, 1H), 7.85 (d, J = 8.1 Hz, 1H), 7.33 (t, J = 7.6 Hz, 1H), 7.21 (t, J = 7.7 Hz, 1H), 6.99 - 6.86 (m, 2H), 5.49 (tt, J = 5.9, 3.0 Hz, 1H), 4.66 (d, J = 5.7 Hz, 2H), 1.99 (d, J = 3.6 Hz, 3H), 1.88 (d, J = 10.6 Hz, 3H), 1.49 - 1.39 (m, 9H).

### Intermediate (1.6a): Synthesis of N-(3'-amino-2,2'-dimethyl-[1,1'-biphenyl]-3-yl)-5-(hydroxymethyl)picolinamide

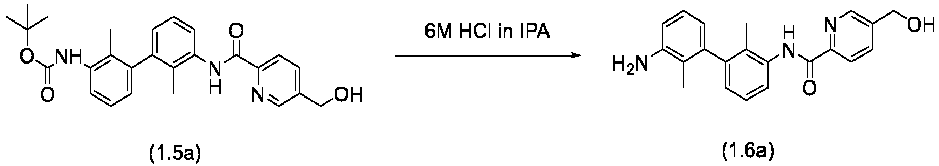

Compound **(1.5a)** (4.2 g, 9.5 mmol, 1 eq) was dissolved in dichloromethane. 6M solution of hydrochloric acid in isopropanol (9.5 mL, 6 mmol, 6 eq) was added. The reaction mixture was stirred at room temperature for 2 hours and extracted with 2M sodium hydroxide and dichloromethane. The organic phase was dried over magnesium sulphate and concentrated to give compound **(1.6a)** (2.7 g, yield 81%). It was used further without purification.

¹H NMR (600 MHz, DMSO) δ 10.29 (s, 1H), 8.66 (dd, J = 2.1, 0.9 Hz, 1H), 8.15 (dd, J = 7.9, 0.8 Hz, 1H), 7.99 (m, 1H), 7.83 (dd, J = 8.1, 1.3 Hz, 1H), 7.26 (t, J = 7.8 Hz, 1H), 7.02 - 6.85 (m, 2H), 6.65 (dd, J = 8.1, 1.3 Hz, 1H), 6.31 (dd, J = 7.5, 1.3 Hz, 1H), 5.51 (t, J = 5.6 Hz, 1H), 4.91 (s, 2H), 4.66 (d, J = 5.6 Hz, 2H), 1.99 (s, 3H), 1.75 (s, 3H).

### Intermediate (1.7a): Synthesis of N-(3'-amino-2,2'-dimethyl-[1,1'-biphenyl]-3-yl)-5-(((tert-butyldimethylsilyl)oxy)methyl)picolinamide

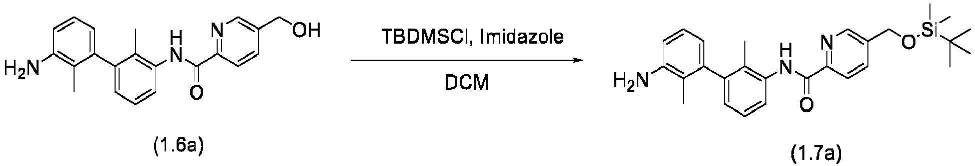

Compound **(1.6a)** (2.7 g, 7.7 mmol, 1 eq) was dissolved in dichloromethane and tetrahydrofuran. Imidazole (1.0 g, 15.5 mmol, 2 eq) and tert-butyldimethylchlorosilane (1.75 g, 11.6 mmol, 1.5 eq) were added. It was stirred at room temperature over the night. Afterwards, the mixture was extracted with dichloromethane and water. The combined organic layer was washed with brine, dried over magnesium sulphate and concentrated. The crude material was used further as compound **(1.7a)** (3.54 g, yield 99%).

¹H NMR (600 MHz, DMSO) δ 10.29 (s, 1H), 8.66 (dd, J = 2.1, 0.9 Hz, 1H), 8.21 - 8.13 (m, 1H), 8.02 - 7.90 (m, 1H), 7.82 (dd, J = 8.1, 1.3 Hz, 1H), 7.26 (t, J = 7.8 Hz, 1H), 6.97 - 6.88 (m, 2H), 6.65 (dd, J = 8.0, 1.3 Hz, 1H), 6.31 (dd, J = 7.4, 1.2 Hz, 1H), 4.90 (d, J = 14.2 Hz, 4H), 1.99 (s, 3H), 1.74 (s, 3H), 0.92 (s, 9H), 0.12 (s, 6H).

### Intermediate (1.8a): Synthesis of 5-bromo-N-(3'-(5-(((tert-butyldimethylsilyl)oxy) methyl)picolinamido)-2,2'-dimethyl-[1,1'-biphenyl]-3-yl)-4-methoxypicolinamide

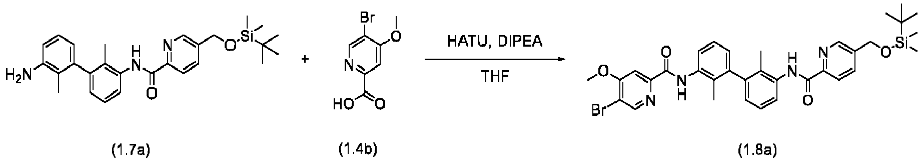

Compound **(1.4b)** (2.7 g, 11.5 mmol, 1.5 eq) was suspended in tetrahydrofuran. N,N Diisopropylethylamine (6.4 mL, 46 mmol, 6 eq) and HATU (3.5 g, 9.2 mmol, 1.2 eq) were added. It was followed by addition of compound **(1.7a)** (3.54 g, 7.7 mmol, 1 eq). The reaction mixture was stirred at room temperature over the night. Afterwards, the mixture was concentrated and extracted with ethyl acetate and water. The organic layer was dried over sodium sulphate and concentrated. The residue was purified by means of flash column chromatography with ethyl acetate and hexane as an eluent to give compound **(1.8a)** (4.7 g, 91% yield).

¹H NMR (600 MHz, DMSO) δ 10.22 (d, J = 3.5 Hz, 2H), 8.62 (s, 1H), 8.55 (dd, J = 2.1, 0.9 Hz, 1H), 8.05 (dd, J = 8.0, 0.8 Hz, 1H), 7.87 (ddt, J = 8.0, 1.9, 0.8 Hz, 1H), 7.80 - 7.65 (m, 3H), 7.21 (td, J = 7.8, 3.9 Hz, 2H), 6.88 (ddd, J = 11.2, 7.6, 1.4 Hz, 2H), 4.77 (s, 2H), 3.96 (s, 3H), 1.89 (d, J = 8.9 Hz, 6H), 0.80 (s, 9H), 0.00 (s, 6H).

### Intermediate (1.9a): Synthesis of N-(3'-(5-(hydroxymethyl)picolinamido)-2,2'-dimethyl-[1,1'-biphenyl]-3-yl)-4-methoxy-5-vinylpicolinamide

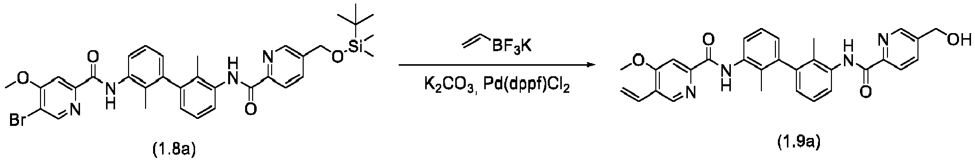

Compound **(1.8a)** (4.7 g, 7.0 mmol, 1 eq) was dissolved in 1,4-dioxane and water (5:2). Potassium carbonate (2.7 g, 20.0 mmol, 2.8 eq) and potassium trifluoro(vinyl)borate (1.4 g, 10.0 mmol, 1.5 eq), Pd(dppf)Cl₂ complex with dichloromethane (0.28 g, 0.35 mmol, 0.05 eq). Argon was bubbled through the reaction mixture. It was stirred at 90°C over the night. Afterwards, the mixture was diluted with ethyl acetate, extracted with water and washed with brine. Organic layer was separated, dried over sodium sulphate and concentrated. The crude material was purified by means of flash column chromatography with ethyl acetate and hexane as an eluent to give compound **(1.9a)** (1.0 g, 29% yield).

¹H NMR (600 MHz, DMSO) δ 10.35 (d, J = 7.0 Hz, 2H), 8.73 (d, J = 10.1 Hz, 1H), 8.66 (dd, J = 2.1, 0.9 Hz, 1H), 8.15 (dd, J = 7.9, 0.9 Hz, 1H), 8.03 - 7.97 (m, 1H), 7.93 (dd, J = 8.1, 1.3 Hz, 1H), 7.90 - 7.86 (m, 1H), 7.80 (d, J = 25.6 Hz, 1H), 7.33 (td, J = 7.8, 5.5 Hz, 2H), 7.04 - 6.96 (m, 2H), 6.91 (dd, J = 17.8, 11.5 Hz, 1H), 6.12 (dd, J = 17.9, 1.4 Hz, 1H), 5.54 - 5.46 (m, 2H), 4.66 (d, J = 5.5 Hz, 2H), 4.03 (s, 3H), 2.02 (d, J = 3.9 Hz, 6H).

### Intermediate (1.10a): Synthesis of N-(3'-(5-formylpicolinamido)-2,2'-dimethyl-[1,1'-biphenyl]-3-yl)-4-methoxy-5-vinylpicolinamide

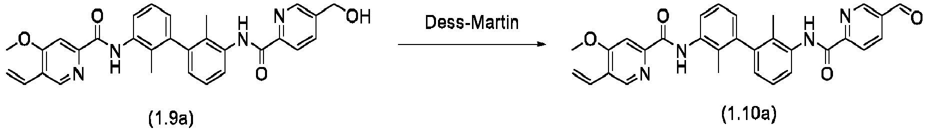

Compound **(1.9a)** (1.0 g, 2 mmol, 1 eq) was dissolved in dichloromethane. Dess-Martin periodinane (1.0 g, 2.45 mmol, 1.2 eq) was added and the reaction mixture stirred at room temperature for 30 minutes. Afterwards, it was quenched with water and extracted with dichloromethane and water. Organic layer was washed with brine and dried over magnesium sulphate and concentrated. The crude material was purified by means of flash column chromatography with ethyl acetate and hexane as an eluent to give compound **(1.10a)** (0.85 g, 69% yield).

¹H NMR (600 MHz, DMSO) δ 10.50 (s, 1H), 10.35 (d, J = 7.6 Hz, 1H), 10.23 (s, 1H), 9.22 (dd, J = 2.0, 0.8 Hz, 1H), 8.73 (d, J = 10.6 Hz, 1H), 8.51 (dd, J = 8.0, 2.0 Hz, 1H), 8.36 (d, J = 8.0 Hz, 1H), 7.93 (dd, J = 8.1, 1.3 Hz, 1H), 7.84 - 7.74 (m, 2H), 7.34 (t, J = 7.8 Hz, 2H), 7.07 - 6.98 (m, 2H), 6.91 (dd, J = 17.9, 11.5 Hz, 1H), 6.12 (dd, J = 17.9, 1.4 Hz, 1H), 5.51 (dd, J = 11.5, 1.4 Hz, 1H), 4.06 - 3.99 (m, 3H), 2.02 (dd, J = 9.4, 6.2 Hz, 6H).

### Intermediate (1.11a): Synthesis of N-(3'-(5-(((2-((tert-butyldimethylsilyl)oxy)ethyl) (methyl)amino)methyl)picolinamido)-2,2'-dimethyl-[1,1'-biphenyl]-3-yl)-4-methoxy-5-vinylpicolinamide

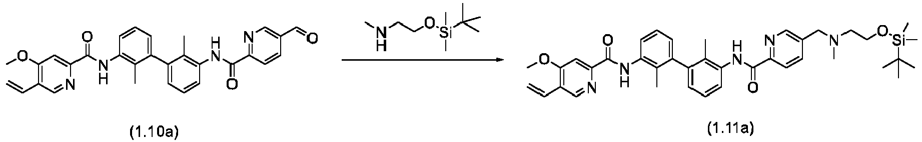

A mixture of compound **(1.10a)** (0.85 g, 1.7 mmol, 1 eq), 2-[[(1,1-dimethylethyl)dimethylsilyl]oxy]-N-methyl-ethanamine (1.27 g, 6.7 mmol, 4 eq.) and two drops of acetic acid in methanol and tetrahydrofuran (1:1) was stirred at 70°C for two hours. Afterwards, the reaction mixture was cooled down to 0°C and sodium triacetoxyborohydride (1.42 g, 6.7 mmol, 4 eq.) was added. Stirring was continued at room temperature over the night, when TLC indicated the completion of the reaction. It was quenched with water and the solvent was concentrated and the residue was extracted with dichloromethane and water. Organic layer was washed with brine and dried over magnesium sulphate and concentrated. The crude material was purified by means of flash column chromatography with ethyl acetate and hexane as an eluent to give compound **(1.11a)** (0.92g, 80% yield).

¹H NMR (600 MHz, DMSO) δ 10.35 (d, J = 8.0 Hz, 2H), 8.73 (d, J = 10.0 Hz, 1H), 8.65 (dd, J = 2.1, 0.9 Hz, 1H), 7.99 (dd, J = 8.0, 2.0 Hz, 1H), 7.93 (dd, J = 8.2, 1.3 Hz, 1H), 7.88 (dt, J = 7.3, 1.9 Hz, 1H), 7.78 (s, 1H), 7.33 (td, J = 7.8, 5.3 Hz, 2H), 7.04 - 6.96 (m, 2H), 6.91 (dd, J = 17.8, 11.5 Hz, 1H), 6.12 (dd, J = 17.9, 1.4 Hz, 1H), 5.51 (dd, J = 11.4, 1.3 Hz, 1H), 4.03 (d, J = 2.5 Hz, 3H), 3.70 (dd, J = 12.4, 6.3 Hz, 4H), 2.23 (s, 3H), 2.02 (d, J = 4.9 Hz, 6H), 0.85 (s, 9H), 0.03 (s, 6H).

### Intermediate (1.12a): Synthesis of N-(3'-(5-(((2-((tert-butyldimethylsilyl)oxy)ethyl) (methyl)amino)methyl)picolinamido)-2,2'-dimethyl-[1,1'-biphenyl]-3-yl)-5-formyl-4-methoxypicolinamide

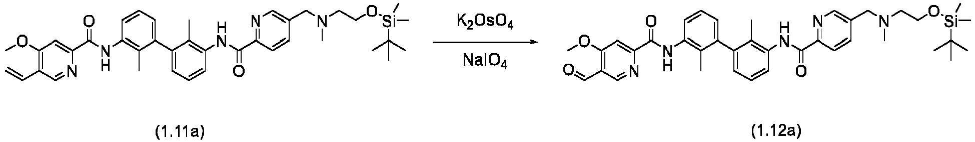

Compound **(1.11a)** (0.92 g, 1.4 mmol, 1 eq) was dissolved in 1,4-dioxane and water (5:1). Potassium osmate dihydrate (25 mg, 0.07 mmol, 0.05 eq) and sodium periodate (0.58 g, 2.7 mmol, 2 eq) were added. Argon was bubbled through the reaction mixture. It was stirred at room temperature for one hour. Afterwards, the mixture was concentrated and extracted with dichloromethane and water. The combined organic layer was washed with brine, dried over magnesium sulphate and concentrated. The crude material was purified by means of flash column chromatography with dichloromethane and ethyl acetate as an eluent to give compound **(1.12a)** (0.56 g, 60%).

¹H NMR (600 MHz, DMSO) δ 10.49 (s, 1H), 10.39 (s, 1H), 10.34 (d, J = 1.8 Hz, 1H), 8.84 (s, 1H), 8.65 (d, J = 2.0 Hz, 1H), 8.14 (d, J = 7.9 Hz, 1H), 7.99 (dd, J = 8.0, 2.1 Hz, 1H), 7.95 (s, 1H), 7.88 (dd, J = 8.2, 1.3 Hz, 1H), 7.83 - 7.76 (m, 1H), 7.33 (q, J = 7.5 Hz, 2H), 7.05 - 6.97 (m, 2H), 4.10 (d, J = 29.9 Hz, 3H), 3.70 (dd, J = 12.3, 6.3 Hz, 4H), 2.23 (s, 3H), 2.05 - 1.99 (m, 6H), 1.33 - 1.16 (m, 2H), 0.85 (s, 9H), 0.03 (s, 6H).

### Intermediate (1.13a - PG): Synthesis N-(3'-(5-(((2-((tert-butyldimethylsilyl)oxy)ethyl) (methyl)amino)methyl)picolinamido)-2,2'-dimethyl-[1,1'-biphenyl]-3-yl)-5-((((1S,2S)-2-hydroxycyclopentyl)amino)methyl)-4-methoxypicolinamide

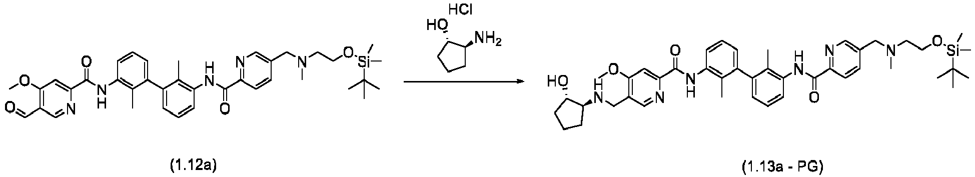

Compound **(1.12a)** (0.16 g, 0.24 mmol, 1 eq), (1R,2R)-2-aminocyclopentanol hydrochloride (0.13 g, 0.9 mmol, 4 eq.), previously mixed with triethylamine (0.13 mL, 0.9 mmol, 4 eq) and two drops of acetic acid were stirred in methanol (4 mL) and tetrahydrofuran (4 mL) at room temperature over the night. Afterwards, the reaction mixture was cooled down to 0°C and lithium borohydride (8 mg, 0.4 mmol, 1.5 eq.) was added. Stirring was continued for five minutes, when TLC indicated the completion of the reaction. It was quenched with water and the solvent was concentrated. The residue was extracted with dichloromethane and water. Organic layer was washed with brine and dried over magnesium sulphate and concentrated. The crude material was purified by means of column chromatography with dichloromethane and ethyl acetate as an eluent, followed by 10% of methanol in ethyl acetate. It allowed to obtain compound **(1.13a** - **PG)** (54 mg, 29% yield).

¹H NMR (600 MHz, DMSO) δ 10.36 (d, J = 19.2 Hz, 2H), 8.65 (d, J = 2.1 Hz, 1H), 8.52 (s, 1H), 8.14 (d, J = 8.0 Hz, 1H), 7.99 (dd, J = 8.0, 2.1 Hz, 1H), 7.92 (d, J = 8.1 Hz, 1H), 7.88 (d, J = 8.4 Hz, 1H), 7.74 (s, 1H), 7.33 (td, J = 7.8, 3.4 Hz, 2H), 6.99 (dt, J = 7.7, 1.7 Hz, 2H), 4.59 - 4.48 (m, 1H), 3.99 (s, 3H), 3.77 (s, 3H), 3.74 - 3.65 (m, 4H), 2.72 (s, 1H), 2.23 (s, 3H), 2.02 (d, J = 3.5 Hz, 6H), 1.90 - 1.76 (m, 2H), 1.57 (dd, J = 12.6, 6.4 Hz, 2H), 1.40 (s, 1H), 1.23 (s, 4H), 0.85 (s, 9H), 0.03 (s, 6H).

### Final compound (R1_C451): 5-((((1S,2S)-2-hydroxycyclopentyl)amino)methyl)-N-(3'-(5-(((2-hydroxyethyl)(methyl)amino)methyl)picolinamido)-2,2'-dimethyl-[1,1'-biphenyl]-3-yl)-4-methoxypicolinamide

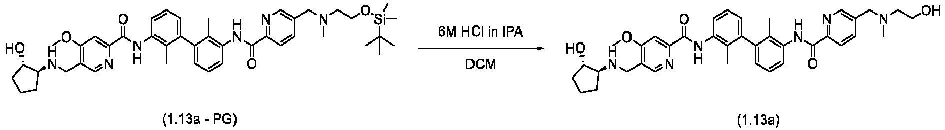

Compound **(1.13a** - **PG)** (54 mg, 0.06 mmol, 1 eq) was dissolved in dichloromethane. 6M solution of hydrochloric acid in isopropanol (0.15 mL, 0.9 mmol, 15 eq) was added. The reaction mixture was stirred at room temperature for 2 hours, when TLC indicated completion of reaction. It was extracted with 1M aqueous solution of sodium hydroxide and dichloromethane, dried over magnesium sulphate and concentrated. The desired product was purified by means of preparative TLC with 30 % of methanol in dichloromethane as an eluent. Obtained material gave compound **(1.13a, R1_C451)** (15 mg, 38% yield) as white solid.

LC/MS: [M-H]⁻= 651.46, 3.93 min, 100% purity

¹H NMR (600 MHz, MeOD) δ 8.71 (d, J = 2.0 Hz, 1H), 8.66 (s, 1H), 8.21 - 8.14 (m, 1H), 8.10 (dd, J = 8.1, 2.1 Hz, 1H), 7.93 - 7.84 (m, 2H), 7.83 (s, 1H), 7.29 - 7.21 (m, 2H), 6.99 - 6.92 (m, 2H), 4.39 - 4.22 (m, 3H), 4.04 (s, 3H), 4.03 - 3.95 (m, 2H), 3.76 (t, J = 5.6 Hz, 2H), 3.29 (s, 1H), 2.84 (t, J = 5.5 Hz, 2H), 2.49 (s, 3H), 2.24 - 2.15 (m, 1H), 1.99 (m, 7H), 1.79 - 1.65 (m, 3H), 1.65 - 1.54 (m, 1H).

### Example 2. Preparation of 5-((((1R,2R)-2-hydroxycyclopentyl)amino)methyl)-N-(3'-(5-(((2-hydroxyethyl)(methyl)amino)methyl)picolinamido)-2,2'-dimethyl-[1,1'-biphenyl]-3-yl)-4-methoxypicolinamide trihydrochloride - Compound (R1_C451_1)

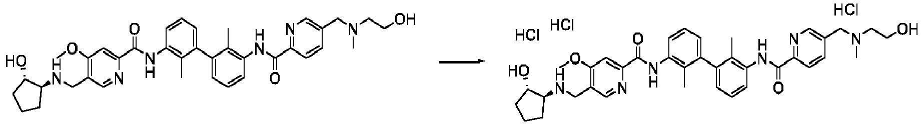

Compound **(R1_C451)** (5.2 mg, 0.01 mmol, 1 eq) was dissolved in methanol. 6M solution of hydrochloric acid in isopropanol (0.013 mL, 0.08 mmol, 10 eq) was added. The reaction mixture was stirred at room temperature for 4 hours. Afterwards, it was concentrated to obtained desired product **(R1_C451_1)** (6 mg, 99% yield) as yellow solid. The material was dried over the night at rotary evaporator.

¹H NMR (600 MHz, DMSO) δ 10.65 (s, 1H), 10.45 (s, 1H), 10.42 (s, 1H), 9.48 (s, 1H), 9.37 (s, 1H), 8.94 (d, J = 2.3 Hz, 1H), 8.73 (s, 1H), 8.32 (dd, J = 8.1, 2.1 Hz, 1H), 8.27 - 8.21 (m, 1H), 7.85 (s, 1H), 7.84 - 7.80 (m, 1H), 7.78 (dd, J = 8.1, 1.3 Hz, 1H), 7.34 (td, J = 7.8, 3.6 Hz, 2H), 7.02 (dt, J = 7.6, 1.5 Hz, 2H), 4.59 (dd, J = 13.4, 4.8 Hz, 1H), 4.47 (dd, J = 13.3, 5.4 Hz, 1H), 4.36 - 4.28 (m, 1H), 4.28 - 4.18 (m, 2H), 4.06 (s, 3H), 3.80 (q, J = 5.9 Hz, 2H), 3.27 - 3.18 (m, 2H), 3.16 (s, 2H), 3.15 - 3.07 (m, 1H), 2.76 (d, J = 4.5 Hz, 3H), 2.16 - 2.05 (m, 1H), 2.02 (d, J = 2.8 Hz, 6H), 1.98 - 1.86 (m, 1H), 1.74 - 1.61 (m, 3H), 1.58 - 1.44 (m, 1H).

### Example 3. Preparation of 5-(((2-hydroxyethyl)(methyl)amino)methyl)-N-(3'-(5-(((2-hydroxyethyl)(methyl)amino)methyl)picolinamido)-2,2'-dimethyl-[1,1'-biphenyl]-3-yl)-4-methoxypicolinamide - Compound (R1_C450)

### Preparation of Intermediates:

### Intermediate (1.11a): N-(3'- {5-[({2-[(tert-butyldimethylsilyl)oxy]ethyl}(methyl)amino) methyl]pyridine-2-amido}-2,2'-dimethyl-[1,1'-biphenyl]-3-yl)-5-ethenyl-4-methoxypyridine-2-carboxamide

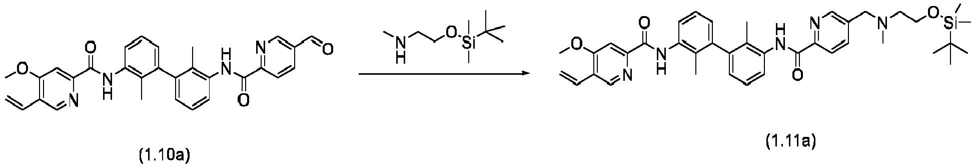

A mixture of intermediate **(1.10a)** (0.85 g, 1.7 mmol, 1 eq), 2-[[(1,1-dimethylethyl)dimethylsilyl]oxy]-N-methyl-ethanamine (1.27 g, 6.7 mmol, 4 eq.) and two drops of acetic acid in methanol and tetrahydrofuran (1:1) was stirred at 70°C for two hours. Afterwards, the reaction mixture was cooled down to 0°C and sodium triacetoxyborohydride (1.42 g, 6.7 mmol, 4 eq.) was added. Stirring was continued at room temperature over the night, when TLC indicated the completion of the reaction. It was quenched with water and the solvent was concentrated and the residue was extracted with dichloromethane and water. Organic layer was washed with brine and dried over magnesium sulphate and concentrated. The crude material was purified by means of flash column chromatography with ethyl acetate and hexane as an eluent to give intermediate **(1.11a)** (0.92g, 80% yield).

¹H NMR (600 MHz, DMSO) δ 10.35 (d, J = 8.0 Hz, 2H), 8.73 (d, J = 10.0 Hz, 1H), 8.65 (dd, J = 2.1, 0.9 Hz, 1H), 7.99 (dd, J = 8.0, 2.0 Hz, 1H), 7.93 (dd, J = 8.2, 1.3 Hz, 1H), 7.88 (dt, J = 7.3, 1.9 Hz, 1H), 7.78 (s, 1H), 7.33 (td, J = 7.8, 5.3 Hz, 2H), 7.04 - 6.96 (m, 2H), 6.91 (dd, J = 17.8, 11.5 Hz, 1H), 6.12 (dd, J = 17.9, 1.4 Hz, 1H), 5.51 (dd, J = 11.4, 1.3 Hz, 1H), 4.03 (d, J = 2.5 Hz, 3H), 3.70 (dd, J = 12.4, 6.3 Hz, 4H), 2.23 (s, 3H), 2.02 (d, J = 4.9 Hz, 6H), 0.85 (s, 9H), 0.03 (s, 6H).

### Intermediate (1.12a): N-(3'- {5-[({2-[(tert-butyldimethylsilyl)oxy]ethyl}(methyl)amino) methyl]pyridine-2-amido}-2,2'-dimethyl-[1,1'-biphenyl]-3-yl)-5-formyl-4-methoxypyridine-2-carboxamide

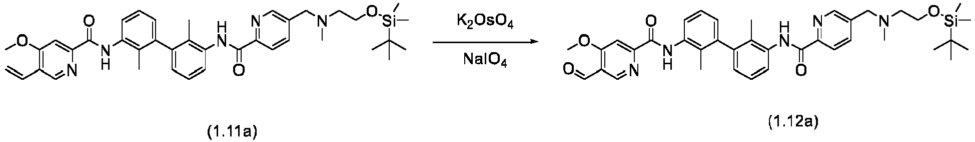

Intermediate **(1.11a)** (0.92 g, 1.4 mmol, 1 eq) was dissolved in 1,4-dioxane and water (5:1). Potassium osmate dihydrate (25 mg, 0.07 mmol, 0.05 eq) and sodium periodate (0.58 g, 2.7 mmol, 2 eq) were added. Argon was bubbled through the reaction mixture. It was stirred at room temperature for one hour. Afterwards, the mixture was concentrated and extracted with dichloromethane and water. The combined organic layer was washed with brine, dried over magnesium sulphate and concentrated. The crude material was purified by means of flash column chromatography with dichloromethane and ethyl acetate as an eluent to give intermediate **(1.12a)** (0.56 g, 60%).

¹H NMR (600 MHz, DMSO) δ 10.49 (s, 1H), 10.39 (s, 1H), 10.34 (d, J = 1.8 Hz, 1H), 8.84 (s, 1H), 8.65 (d, J = 2.0 Hz, 1H), 8.14 (d, J = 7.9 Hz, 1H), 7.99 (dd, J = 8.0, 2.1 Hz, 1H), 7.95 (s, 1H), 7.88 (dd, J = 8.2, 1.3 Hz, 1H), 7.83 - 7.76 (m, 1H), 7.33 (q, J = 7.5 Hz, 2H), 7.05 - 6.97 (m, 2H), 4.10 (d, J = 29.9 Hz, 3H), 3.70 (dd, J = 12.3, 6.3 Hz, 4H), 2.23 (s, 3H), 2.05 - 1.99 (m, 6H), 1.33 - 1.16 (m, 2H), 0.85 (s, 9H), 0.03 (s, 6H).

### Intermediate (1.13a - PG): N-(3'-{5-[({2-[(tert-butyldimethylsilyl)oxy]ethyl}(methyl) amino)methyl]pyridine-2-amido}-2,2'-dimethyl-[1,1'-biphenyl]-3-yl)-5-{[(2-hydroxyethyl)(methyl)amino]methyl}-4-methoxypyridine-2-carboxamide

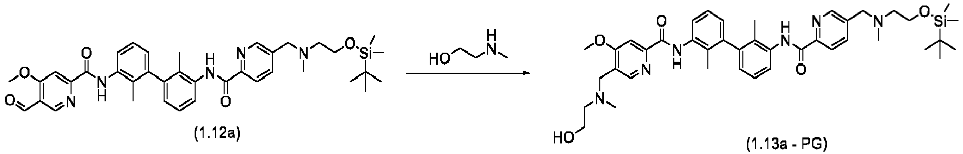

Intermediate **(1.12a)** (0.163 g, 0.24 mmol, 1 eq), 2-(methylamino)ethanol (0.076 mL, 0.96 mmol, 4 eq.) and two drops of acetic acid were stirred in MeOH (4 mL) and THF (4 mL) at room temperature for 3 hours. Afterwards, the reaction mixture was cooled down to 0°C and sodium triacetoxyborohydride (203 mg, 0.96 mmol, 4 eq.) was added. Stirring was continued over the night, when TLC indicated the completion of the reaction. It was quenched with water and the solvent was concentrated. The residue was extracted with dichloromethane and water. Organic layer was washed with brine and dried over magnesium sulphate and concentrated. The crude material was purified by means of column chromatography with dichloromethane and ethyl acetate as an eluent, followed by 90% of methanol in ethyl acetate. It allowed to obtain intermediate **(1.13a** - **PG)** (33 mg, 18% yield).

¹H NMR (600 MHz, DMSO) δ 10.35 (s, 1H), 10.31 (s, 1H), 8.62 (dd, J = 2.1, 0.8 Hz, 1H), 8.51 (s, 1H), 8.11 (dd, J = 7.9, 0.8 Hz, 1H), 7.96 (dd, J = 8.0, 2.1 Hz, 1H), 7.87 (ddd, J = 13.2, 8.1, 1.3 Hz, 2H), 7.72 (s, 1H), 7.30 (td, J = 7.8, 2.0 Hz, 2H), 6.97 (dt, J = 7.6, 1.6 Hz, 2H), 4.39 (t, J = 5.4 Hz, 1H), 3.95 (s, 3H), 3.68 (t, J = 6.1 Hz, 2H), 3.66 (s, 2H), 3.57 (s, 2H), 3.53 - 3.46 (m, 2H), 2.44 (t, J = 6.3 Hz, 2H), 2.19 (d, J = 12.1 Hz, 6H), 1.99 (d, J = 3.2 Hz, 6H), 1.21 (s, 2H), 0.82 (s, 9H), 0.00 (s, 6H).

### Final compound (R1_C450): 5-(((2-hydroxyethyl)(methyl)amino)methyl)-N-(3'-(5-(((2-hydroxyethyl)(methyl)amino)methyl)picolinamido)-2,2'-dimethyl-[1,1'-biphenyl]-3-yl)-4-methoxypicolinamide

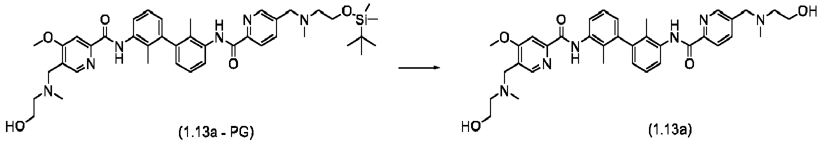

Intermediate **(1.13a** - **PG)** (40 mg, 0.04 mmol, 1 eq) was dissolved in dichloromethane. 6M solution of hydrochloric acid in isopropanol (0.11 mL, 0.7 mmol, 15 eq) was added. The reaction mixture was stirred at room temperature for 2 hours, when TLC indicated completion of reaction. It was extracted with 1M aqueous solution of sodium hydroxide and dichloromethane, dried over magnesium sulphate and concentrated. The desired product was purified by means of preparative TLC with 30 % of methanol in dichloromethane as an eluent. Obtained material gave desired product **(1.13a, R1_C450)** (13 mg, 47% yield).

¹H NMR (600 MHz, MeOD) δ 8.65 (d, J = 2.1 Hz, 1H), 8.49 (s, 1H), 8.17 (dd, J = 8.0, 0.8 Hz, 1H), 8.00 (dd, J = 8.0, 2.1 Hz, 1H), 7.94 (ddd, J = 16.9, 8.1, 1.3 Hz, 2H), 7.82 (s, 1H), 7.31 (t, J = 7.8 Hz, 2H), 7.02 (dt, J = 7.6, 1.5 Hz, 2H), 4.00 (s, 3H), 3.76 - 3.63 (m, 8H), 2.60 (dt, J = 9.6, 6.0 Hz, 4H), 2.28 (d, J = 4.1 Hz, 6H), 2.08 (s, 6H).

LCMS: [[M-H]⁻+2Na⁺]= 671.26, 100%, 3.72 min

### Example 4. Preparation of (S)-N-(3'-(5-(((2-hydroxyethyl)(methyl)amino)methyl)picolinamido)-2,2'-dimethyl-[1,1'-biphenyl]-3-yl)-5-(((1-hydroxypropan-2-yl)amino)methyl)-4-methoxypicolinamide - Compound (R1_C458)

### Preparation of Intermediates:

### Intermediate (1.13a - PG): N-(3'-{5-[({2-[(tert-butyldimethylsilyl)oxy]ethyl}(methyl) amino)methyl]pyridine-2-amido}-2,2'-dimethyl-[1,1'-biphenyl]-3-yl)-5-({[(1S,2S)-2-hydroxycyclopentyl]amino}methyl)-4-methoxypyridine-2-carboxamide

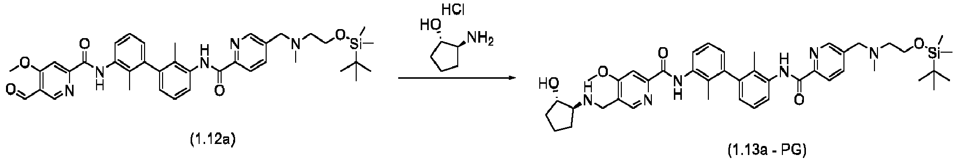

Intermediate **(1.12a)** (0.14 g, 0.2 mmol, 1 eq), (S)-2-aminopropan-1-ol (0.13 mL, 1.65 mmol, 8 eq.) and two drops of acetic acid were stirred in MeOH (4 mL) and THF (4 mL) at room temperature for two hours. Afterwards, the reaction mixture was cooled down to 0°C and sodium cyanoborohydride (0.1 g, 1.65 mmol, 8 eq.) was added. Stirring was continued for two hours, when TLC indicated the completion of the reaction. It was quenched with water and the solvent was concentrated. The crude material was purified by means of column chromatography with dichloromethane and ethyl acetate as an eluent, followed by 90% of methanol in ethyl acetate. It allowed to obtain intermediate **(1.13a** - **PG)** (53 mg, 34% yield).

¹H NMR (600 MHz, MeOD) δ 8.69 - 8.63 (m, 1H), 8.53 (s, 1H), 8.17 (dd, J = 8.0, 0.8 Hz, 1H), 8.01 - 7.91 (m, 3H), 7.89 (s, 1H), 7.35 - 7.28 (m, 2H), 7.05 - 6.99 (m, 2H), 4.22 - 4.10 (m, 2H), 4.08 (s, 3H), 3.84 - 3.74 (m, 4H), 3.74 - 3.65 (m, 2H), 3.63 - 3.48 (m, 2H), 2.61 (t, J = 5.8 Hz, 2H), 2.07 (d, J = 3.1 Hz, 6H), 1.26 (d, J = 6.7 Hz, 4H), 0.89 (s, 9H), 0.06 (s, 6H).

### Final compound (R1_C458): (S)-N-(3'-(5-(((2-hydroxyethyl)(methyl)amino)methyl)picolinamido)-2,2'-dimethyl-[1,1'-biphenyl]-3-yl)-5-(((1-hydroxypropan-2-yl)amino)methyl)-4-methoxypicolinamide

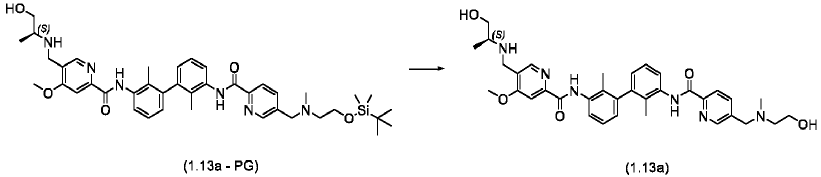

Intermediate **(1.13a** - **PG)** (53 mg, 0.07 mmol, 1 eq) was dissolved in dichloromethane. 6M solution of hydrochloric acid in isopropanol (0.18 mL, 1.0 mmol, 15 eq) was added. The reaction mixture was stirred at room temperature for 2 hours, when TLC indicated completion of reaction. It was concentrated. The desired product was purified by means of preparative TLC with 30 % of methanol in dichloromethane as an eluent. Obtained material gave desired product (10 mg, 22% yield).

¹H NMR (600 MHz, MeOD) δ 8.73 - 8.68 (m, 1H), 8.60 (s, 1H), 8.21 (d, J = 8.0 Hz, 1H), 8.05 (dd, J = 8.0, 2.1 Hz, 1H), 7.96 - 7.89 (m, 3H), 7.38 - 7.31 (m, 2H), 7.09 - 7.02 (m, 2H), 4.31 - 4.20 (m, 2H), 4.11 (s, 3H), 3.79 (d, J = 12.9 Hz, 3H), 3.72 (t, J = 5.9 Hz, 2H), 3.62 - 3.55 (m, 1H), 3.28 - 3.21 (m, 1H), 2.66 (t, J = 5.8 Hz, 2H), 2.34 (s, 3H), 2.10 (d, J = 2.8 Hz, 6H), 1.31 (d, J = 6.6 Hz, 3H).

LCMS: [M-H]⁻= 625.27, 100%, 3.9 min

### Synthesis of compounds, where R₃ = R₄ = -OMe:

### Example 5. Preparation of 5-((((1S,2S)-2-hydroxycyclopentyl)amino)methyl)-N-(3'-(5-((((S)-1-hydroxypropan-2-yl)amino)methyl)-4-methoxypicolinamido)-2,2'-dimethyl-[1,1'-biphenyl]-3-yl)-4-methoxypicolinamide - Compound (R1_C428)

### Preparation of Intermediates:

### Intermediate (1.4b): Synthesis of 5-bromo-4-methoxypicolinic acid

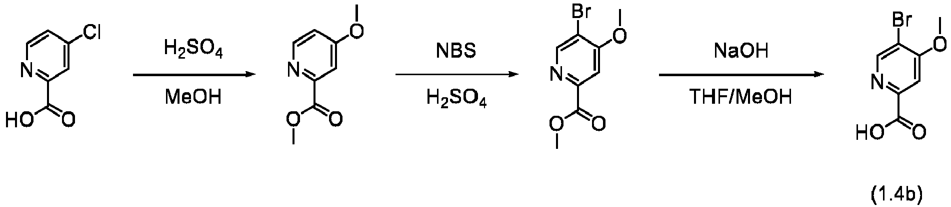

4-Chloropicolinic acid (10.0 g, 6.3 mol, 1 eq) was dissolved in anhydrous methanol (300 ml). Concentrated sulfuric acid was added (2 mL). The reaction mixture was stirred for three days at 60°C. Afterwards, it was concentrated and extracted with sodium bicarbonate and dichloromethane. Combined organic layer was dried over magnesium sulphate and concentrated. The crude material was used further without any purification (9.23 g, 87% yield).

¹H NMR (600 MHz, DMSO) δ 8.52 (d, J = 5.6 Hz, 1H), 7.55 (d, J = 2.6 Hz, 1H), 7.22 (dd, J = 5.6, 2.6 Hz, 1H), 3.90 (s, 4H), 3.87 (s, 3H).

Methyl 4-methoxypyridine-2-carboxylate (17.6 g, 0.1 mol, 1 eq) was dissolved in concentrated sulfuric acid (35 mL), N-bromosuccinimide (20.6 g, 0.11 mol, 1.1 eq) was added. The reaction mixture was stirred at room temperature over the night. Afterwards, it was carefully quenched with aqueous solutions of sodium bicarbonate and sodium thiosulphate. The mixture was extracted with dichloromethane. Organic layer was separated, dried over magnesium sulphate and concentrated. The crude material was crystallized (3.9 g) and the residue was purified by means of flash column chromatography with acetone and hexane as an eluent to give compound 5-bromo-4-methoxy-methyl ester-2-pyridinecarboxylic acid, (7.57 g, 29% yield).

¹H NMR (600 MHz, DMSO) δ 8.70 (s, 1H), 7.70 (s, 1H), 4.03 (s, 3H), 3.89 (s, 3H).

5-bromo-4-methoxy-methyl ester-2-pyridinecarboxylic acid (7.59 g, 30 mmol, 1 eq) was dissolved in tetrahydrofuran and methanol (1:1). Aqueous solution of sodium hydroxide was added (1.23 g, 30 mmol, 1 eq, 20 mL). The reaction mixture was stirred for 30 min at room temperature. Afterwards, the reaction mixture was concentrated, acidified to pH 6-7 by means of concentrated hydrochloric acid and extracted with dichloromethane and methanol and small amount of water. The combined organic layer was dried over magnesium sulphate and concentrated. Compound **(1.4b))** was obtained (2.75 g, 33% yield) as a white solid.

¹H NMR (600 MHz, DMSO) δ 8.68 (s, 1H), 7.69 (s, 1H), 4.02 (s, 3H).

### Intermediate (1.5b): Synthesis of tert-butyl (3'-(5-bromo-4-methoxypicolinamido)-2,2'-dimethyl-[1,1'-biphenyl]-3-yl)carbamate

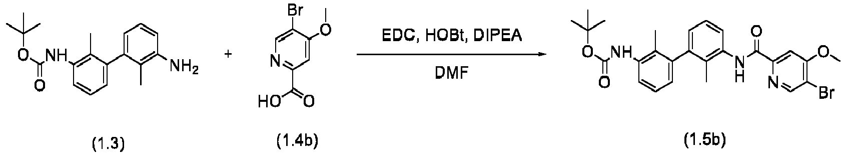

Compound **(1.4b)** (2.4 g, 10.5 mmol, 1.2 eq) was suspended in N,N-dimethylformamide (50 mL). N,N-Diisopropylethylamine (6.9 mL, 39 mmol, 4.5 eq), EDC HCl (3.36 g, 17.5 mmol, 2 eq) and HOBt hydrate (2.68 g, 17.5 mmol, 2 eq) were added. It was followed by addition of compound **(1.3)** (2.74 g, 9 mmol, 1 eq). The reaction mixture was stirred at room temperature over the night. Afterwards, the mixture was diluted with water and extracted with dichloromethane. Organic layer was separated, dried over magnesium sulphate and concentrated. The crude material was purified by means of flash column chromatography with ethyl acetate and hexane as an eluent to give compound **(1.5b)** (3.4 g, 73% yield).

¹H NMR (300 MHz, DMSO) δ 10.30 (s, 1H), 8.72 (s, 1H), 8.58 (s, 1H), 7.96 - 7.69 (m, 2H), 7.40 - 7.15 (m, 3H), 7.05 - 6.84 (m, 2H), 4.07 (s, 3H), 1.92 (d, J = 20.8 Hz, 6H), 1.46 (s, 9H).

### Intermediate (1.6b): Synthesis of tert-butyl (3'-(4-methoxy-5-vinylpicolinamido)-2,2'-dimethyl-[1,1'-biphenyl]-3-yl)carbamate

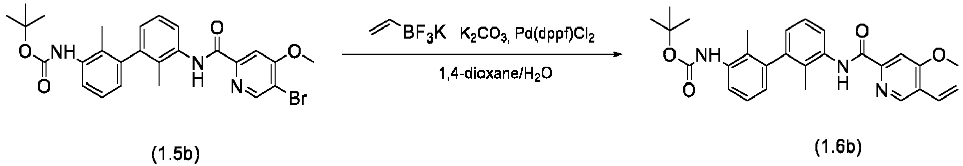

Compound **(1.5b)** (3.4 g, 6.5 mmol, 1 eq) was dissolved in mixture of 1,4-dioxane and water (5:2). Potassium carbonate (3.57 g, 25.8 mmol, 4 eq) and potassium trifluoro(vinyl)borate (1.2 g, 9 mmol, 1.4 eq), Pd(dppf)Cl₂ (0.33 g, 0.45 mmol, 0.07 eq). Argon was bubbled through the reaction mixture. It was stirred at 90°C over the night. Afterwards, the mixture was diluted with ethyl acetate, extracted with water and washed with brine. Organic layer was separated, dried over sodium sulphate and concentrated. The crude material was purified by means of flash column chromatography with acetone and hexane as an eluent to give compound (1.6b) (2.49 g, 81% yield).

¹H NMR (600 MHz, DMSO) δ 10.34 (s, 1H), 8.71 (s, 1H), 8.61 (s, 1H), 7.89 (dd, J = 8.1, 1.3 Hz, 1H), 7.77 (s, 1H), 7.39 - 7.26 (m, 2H), 7.21 (t, J = 7.7 Hz, 1H), 6.97 - 6.84 (m, 3H), 6.12 (dd, J = 17.9, 1.4 Hz, 1H), 5.51 (dd, J = 11.4, 1.3 Hz, 1H), 4.03 (d, J = 4.0 Hz, 4H), 1.98 (s, 3H), 1.89 (s, 3H), 1.46 (s, 9H).

### Intermediate (1.7b): Synthesis of N-(3'-amino-2,2'-dimethyl-[1,1'-biphenyl]-3-yl)-4-methoxy-5-vinylpicolinamide dihydrochloride

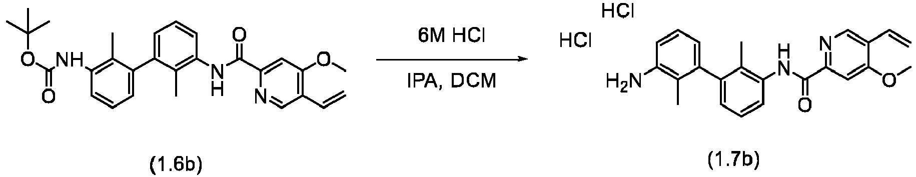

Compound **(1.6b)** (2.49 g, 5.26 mmol, 1 eq) was dissolved in dichloromethane. 6M solution of hydrochloric acid in isopropanol (7.5 mL, 44 mmol, 8.5 eq) was added. The reaction mixture was stirred at room temperature for 5 hours and evaporated to give compound **(1.7b)** (2.4 g, yield 100%). It was used further without purification.

¹H NMR (300 MHz, DMSO) δ 10.37 (s, 1H), 8.70 (s, 1H), 7.98 - 7.84 (m, 1H), 7.79 (s, 1H), 7.53 - 7.26 (m, 3H), 7.14 (d, J = 7.6 Hz, 1H), 7.05 - 6.81 (m, 2H), 6.11 (dd, J = 17.9, 1.4 Hz, 1H), 5.52 (dd, J = 11.4, 1.4 Hz, 1H), 4.03 (s, 3H), 2.03 (s, 3H), 1.96 (s, 3H).

### Intermediate (1.8b): Synthesis of 5-bromo-4-methoxy-N-(3'-(4-methoxy-5-vinylpicolinamido)-2,2'-dimethyl-[1,1'-biphenyl]-3-yl)picolinamide

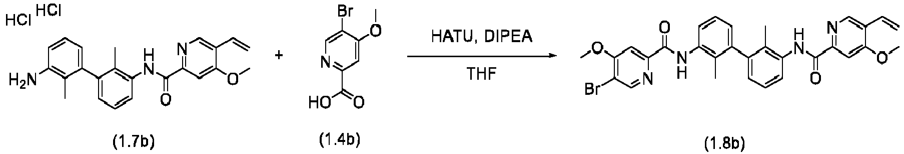

Compound **(1.4b)** (1.27 g, 5.5 mmol, 1 eq) was suspended in tetrahydrofuran. N,N-Diisopropylethylamine (2.9 mL, 16 mmol, 3 eq) and HATU (2.49 g, 6.57 mmol, 1.2 eq) were added. It was followed by addition of compound **(1.7b)** (2.44 g, 5.5 mmol, 1 eq). The reaction mixture was stirred at room temperature over the night. Afterwards, the mixture was concentrated and purified by means of flash column chromatography with ethyl acetate and hexane as an eluent to give compound **(1.8b)** and some impurities (4.9 g).

¹H NMR (300 MHz, DMSO) δ 10.33 (d, J = 4.6 Hz, 2H), 8.80 - 8.66 (m, 2H), 7.93 (d, J = 8.0 Hz, 1H), 7.80 (d, J = 13.3 Hz, 3H), 7.33 (t, J = 7.8 Hz, 2H), 7.05 - 6.81 (m, 3H), 6.12 (dd, J = 17.8, 1.4 Hz, 1H), 5.51 (dd, J = 11.5, 1.4 Hz, 1H), 4.08 (s, 3H), 4.03 (s, 3H), 2.01 (d, J = 6.2 Hz, 6H).

### Intermediate (1.9b): Synthesis of 5-bromo-N-(3'-(5-formyl-4-methoxypicolinamido)-2,2'-dimethyl-[1,1'-biphenyl]-3-yl)-4-methoxypicolinamide

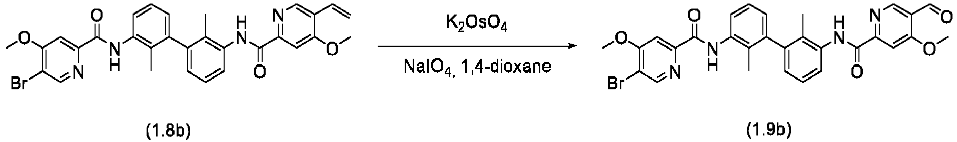

Compound **(1.8b)** (4.9 g (impure mixture), 5.17 mmol, 1 eq) was dissolved in 1,4-dioxane and water (5:1). Potassium osmate dihydrate (38 mg, 0.1 mmol, 0.02 eq) and Sodium periodate (2.2 g, 10 mmol, 2 eq) were added. Argon was bubbled through the reaction mixture. It was stirred at room temperature over the night. Afterwards, the mixture was concentrated and extracted with dichloromethane and water. The combined organic layer was washed with brine, dried over magnesium sulphate and concentrated. The crude material was purified by means of flash column chromatography with ethyl acetate and hexane as an eluent to give compound (1.9b) (2.56 g, 84% yield).

¹H NMR (300 MHz, DMSO) δ 10.56 - 10.24 (m, 3H), 8.84 (d, J = 1.1 Hz, 1H), 8.74 (d, J = 1.1 Hz, 1H), 7.95 (s, 1H), 7.90 - 7.73 (m, 3H), 7.34 (t, J = 7.7 Hz, 2H), 7.02 (dd, J = 7.5, 3.5 Hz, 2H), 4.13 (d, J = 1.1 Hz, 3H), 4.08 (d, J = 1.1 Hz, 3H), 2.00 (s, 6H).

### Intermediate (1.10b): Synthesis of 5-formyl-4-methoxy-N-(3'-(4-methoxy-5-vinylpicolinamido) -2,2' -dimethyl- [1,1' -biphenyl] -3 -yl)picolinamide

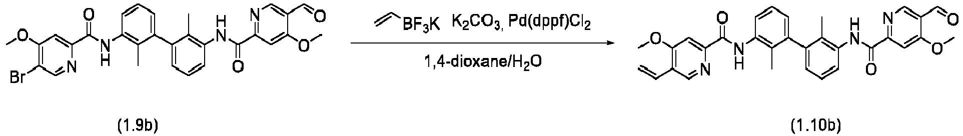

Compound **(1.9b)** (2.56 g, 4.3 mmol, 1 eq) was dissolved in 1,4-dioxane and water (5:2). Potassium carbonate (1.8 g, 13 mmol, 3 eq) and potassium trifluoro(vinyl)borate (0.64 g, 4.8 mmol, 1.1 eq), Pd(dppf)Cl₂ (0.16 g, 0.22 mmol, 0.05 eq). Argon was bubbled through the reaction mixture. It was stirred at 90°C over the weekend. Afterwards, the mixture was diluted with ethyl acetate, extracted with water and washed with brine. Organic layer was separated, dried over sodium sulphate and concentrated. The crude material was purified by means of flash column chromatography with ethyl acetate and hexane as an eluent to give compound **(1.10b)** (0.936 g, 40% yield).

¹H NMR (600 MHz, DMSO) δ 10.49 (s, 1H), 10.39 (s, 1H), 10.35 (s, 1H), 8.84 (s, 1H), 8.72 (s, 1H), 7.98 - 7.88 (m, 2H), 7.84 - 7.73 (m, 2H), 7.34 (td, J = 7.8, 1.9 Hz, 2H), 7.08 - 6.96 (m, 2H), 6.91 (dd, J = 17.8, 11.5 Hz, 1H), 6.12 (dd, J = 17.8, 1.4 Hz, 1H), 5.51 (dd, J = 11.4, 1.3 Hz, 1H), 4.13 (s, 3H), 4.03 (s, 3H), 2.02 (d, J = 9.9 Hz, 6H).

### Intermediate (1.11b): Synthesis of (S)-5-(((1-hydroxypropan-2-yl)amino)methyl)-4-methoxy-N-(3'-(4-methoxy-5-vinylpicolinamido)-2,2'-dimethyl-[1,1'-biphenyl]-3-yl)picolinamide

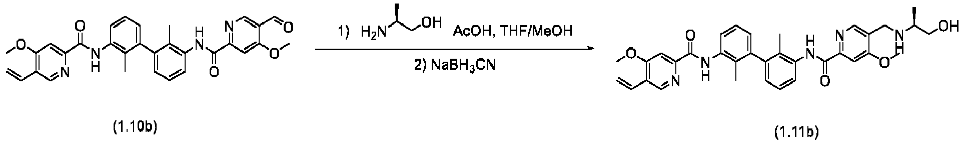

A mixture of compound **(1.10b)** (0.936 g, 1.74 mmol, 1 eq), (S)-2-aminopropan-1-ol (0.68 mL, 8.72 mmol, 5 eq.) and two drops of acetic acid in methanol and tetrahydrofuran (1:1) was stirred at 70°C for four hours. Afterwards, the reaction mixture was cooled down to 0°C and sodium cyanoborohydride (0.548 g, 8.72 mmol, 5 eq.) was added. Stirring was continued at room temperature over the night, when TLC indicated the completion of the reaction. It was quenched with water and the solvent was concentrated and the residue was extracted with dichloromethane and water. Organic layer was washed with brine and dried over magnesium sulphate and concentrated. The crude material was used further without any other purification as compound **(1.11b)** (0.953g, 91% yield).

LC/MS: [M-H]⁺ = 596.36, 100% purity, 6.47 min

¹H NMR (600 MHz, DMSO) δ 10.37 (d, J = 10.5 Hz, 2H), 8.73 (s, 1H), 8.54 (s, 1H), 7.99 - 7.88 (m, 2H), 7.77 (d, J = 22.5 Hz, 2H), 7.34 (td, J = 7.8, 2.7 Hz, 2H), 7.00 (dt, J = 7.5, 1.8 Hz, 2H), 6.91 (dd, J = 17.9, 11.5 Hz, 1H), 6.13 (dd, J = 17.9, 1.4 Hz, 1H), 5.52 (dd, J = 11.4, 1.4 Hz, 1H), 4.54 (t, J = 5.5 Hz, 1H), 4.04 (s, 3H), 4.00 (s, 3H), 3.87 - 3.73 (m, 2H), 3.31 - 3.21 (m, 2H), 2.60 - 2.54 (m, 1H), 2.03 (d, J = 2.2 Hz, 6H), 0.95 (d, J = 6.3 Hz, 3H).

### Intermediate (1.11b - PG): Synthesis of (S)-5-(((1-((tert-butyldimethylsilyl)oxy)propan-2-yl)amino)methyl)-4-methoxy-N-(3'-(4-methoxy-5-vinylpicolinamido)-2,2'-dimethyl-[1,1'-biphenyl]-3-yl)picolinamide

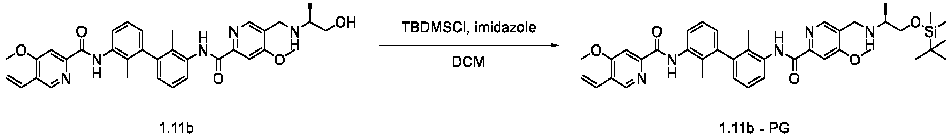

Compound **(1.11b)** (0.953 g, 1.6 mmol, 1 eq) was dissolved in dichloromethane. Imidazole (0.34 g, 4.9 mmol, 3.1 eq) and tert-butyldimethylchlorosilane (0.5 g, 3.3 mmol, 2.1 eq) were added. It was stirred at room temperature over the night. Afterwards, the mixture was extracted with dichloromethane and water. The combined organic layer was washed with brine, dried over magnesium sulphate and concentrated. The crude material was purified by means of flash column chromatography with ethyl acetate and hexane followed by methanol (up to 10%) as an eluent to give compound **(1.11b** - **PG)** (0.82 g, yield 72%).

¹H NMR (600 MHz, DMSO) δ 10.36 (d, J = 9.1 Hz, 2H), 8.72 (s, 1H), 8.51 (s, 1H), 7.93 (ddd, J = 7.9, 6.4, 1.3 Hz, 2H), 7.76 (d, J = 21.8 Hz, 2H), 7.33 (td, J = 7.8, 2.3 Hz, 2H), 6.99 (ddd, J = 7.6, 2.8, 1.3 Hz, 2H), 6.97 - 6.85 (m, 1H), 6.12 (dd, J = 17.8, 1.4 Hz, 1H), 5.51 (dd, J = 11.4, 1.3 Hz, 1H), 4.07 - 4.00 (m, 3H), 3.98 (s, 3H), 3.90 - 3.71 (m, 2H), 3.48 - 3.36 (m, 2H), 2.59 (d, J = 6.3 Hz, 1H), 2.02 (d, J = 3.6 Hz, 6H), 1.17 (t, J = 7.1 Hz, 1H), 0.96 (d, J = 6.3 Hz, 3H), 0.84 (s, 9H), 0.00 (d, J = 7.4 Hz, 6H).

### Intermediate (1.12b): Synthesis of (S)-5-(((1-((tert-butyldimethylsilyl)oxy)propan-2-yl)amino)methyl)-N-(3'-(5-formyl-4-methoxypicolinamido)-2,2'-dimethyl-[1,1'-biphenyl]-3-yl)-4-methoxypicolinamide

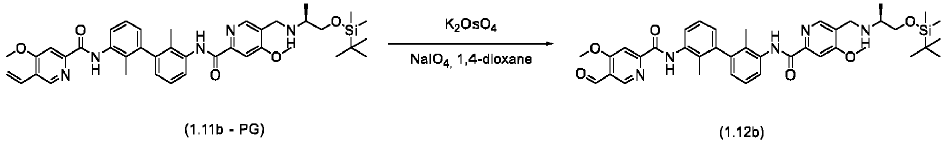

Compound **(1.11b** - **PG)** (0.82 g, 1.15 mmol, 1 eq) was dissolved in 1,4-dioxane and water (5:1). Potassium osmate dihydrate (21 mg, 0.06 mmol, 0.05 eq) and sodium periodate (0.74 g, 3.4 mmol, 3 eq) were added. Argon was bubbled through the reaction mixture. It was stirred at room temperature for 3 hours. Afterwards, the mixture was concentrated and extracted with dichloromethane and water. The combined organic layer was washed with brine, dried over magnesium sulphate and concentrated. The crude material was purified by means of flash column chromatography with dichloromethane and ethyl acetate as an eluent to give compound **(1.12b)** (0.548 g, 66%) as brown solid.

LC/MS: [M-H]⁺ = 712.47, 98% purity, 7.7 min

¹H NMR (600 MHz, DMSO) δ 10.49 (s, 1H), 10.39 (s, 1H), 10.37 (s, 1H), 8.84 (s, 1H), 8.51 (s, 1H), 7.95 (s, 1H), 7.92 (dd, J = 8.1, 1.3 Hz, 1H), 7.79 (dd, J = 8.1, 1.3 Hz, 1H), 7.74 (s, 1H), 7.34 (m, 2H), 7.05 - 6.97 (m, 2H), 4.13 (s, 3H), 3.98 (s, 3H), 3.89 - 3.69 (m, 2H), 3.49 - 3.38 (m, 2H), 2.65 - 2.55 (m, 1H), 2.01 (d, J = 6.4 Hz, 6H), 0.96 (d, J = 6.3 Hz, 3H), 0.84 (s, 9H), 0.00 (d, J = 7.4 Hz, 6H).

### Intermediate (1.13b - PG): Synthesis of 5-((((S)-1-((tert-butyldimethylsilyl)oxy)propan-2-yl)amino)methyl)-N-(3'-(5-((((1S,2S)-2-hydroxycyclopentyl)amino)methyl)-4-methoxypicolinamido)-2,2'-dimethyl-[1,1'-biphenyl]-3-yl)-4-methoxypicolinamide

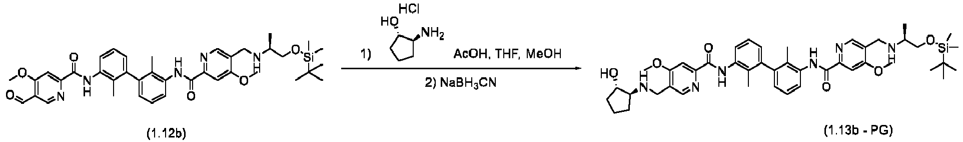

Compound **(1.12b)** (0.35 g, 0.5 mmol, 1 eq), (1R,2R)-2-aminocyclopentanol hydrochloride (0.27 g, 2.0 mmol, 4 eq.), previously mixed with triethylamine (0.27 mL, 2.0 mmol, 4 eq) and two drops of acetic acid were stirred in MeOH (4 mL) and THF (4 mL) at room temperature over the night. Afterwards, the reaction mixture was cooled down to 0°C and sodium cyanoborohydride (0.12 g, 2.0 mmol, 4 eq.) was added. Stirring was continued at room temperature for one hour, when TLC indicated the completion of the reaction. It was quenched with water and the solvent was concentrated. The residue was extracted with dichloromethane and water. Organic layer was washed with brine and dried over magnesium sulphate and concentrated. The crude material was purified by means of column chromatography with dichloromethane and ethyl acetate as an eluent, followed by 10% of methanol in ethyl acetate. It allowed to obtain compound **(1.13b** - **PG)** (0.229 g, 58% yield).

LC/MS: [M-H]⁺ = 797.55, 100% purity, 6.4 min

¹H NMR (600 MHz, DMSO) δ 10.37 (d, J = 2.1 Hz, 2H), 8.51 (d, J = 4.9 Hz, 2H), 7.96 - 7.89 (m, 2H), 7.74 (d, J = 1.6 Hz, 2H), 7.33 (t, J = 7.8 Hz, 2H), 6.99 (dd, J = 7.6, 1.3 Hz, 2H), 4.58 - 4.50 (m, 1H), 3.98 (d, J = 7.3 Hz, 6H), 3.81 - 3.72 (m, 4H), 3.50 - 3.37 (m, 3H), 2.78 - 2.69 (m, 1H), 2.68 - 2.55 (m, 2H), 2.02 (d, J = 2.4 Hz, 6H), 1.91 - 1.75 (m, 2H), 1.65 - 1.52 (m, 2H), 1.45 - 1.33 (m, 1H), 1.33 - 1.25 (m, 1H), 1.23 (s, 1H), 0.96 (d, J = 6.3 Hz, 3H), 0.84 (s, 9H), 0.00 (d, J = 7.4 Hz, 6H).

### Final compound (R1_C428): Synthesis of 5-((((1S,2S)-2-hydroxycyclopentyl)amino) methyl)-N-(3'-(5-((((S)-1-hydroxypropan-2-yl)amino)methyl)-4-methoxypicolinamido)-2,2'-dimethyl-[1,1'-biphenyl]-3-yl)-4-methoxypicolinamide

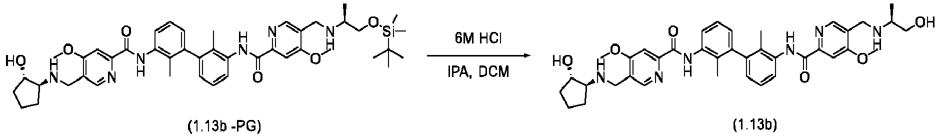

Compound **(1.13b** - **PG)** (60 mg, 0.08 mmol, 1 eq) was dissolved in dichloromethane. 6M solution of hydrochloric acid in isopropanol (0.125 mL, 0.75 mmol, 10 eq) was added. The reaction mixture was stirred at room temperature over the night, when TLC indicated completion of reaction. It was extracted with 1M aqueous solution of sodium hydroxide, dried over magnesium sulphate and concentrated. The desired product was purified by means of preparative TLC with 30 % of methanol in dichloromethane as an eluent. Obtained material gave compound **(1.13b, R1_C428)** (18 mg, 35% yield) as white solid.

LC/MS: [M+H]⁺ = 683.49; 4.01 min, 98.15% purity

¹H NMR (600 MHz, MeOD) δ 8.48 (d, J = 5.9 Hz, 2H), 7.93 (d, J = 8.2 Hz, 2H), 7.85 (d, J = 7.4 Hz, 2H), 7.31 (t, J = 7.8 Hz, 2H), 7.02 (d, J = 7.6 Hz, 2H), 4.05 (d, J = 4.8 Hz, 6H), 4.00 - 3.92 (m, 4H), 3.63 - 3.57 (m, 1H), 3.45 - 3.39 (m, 1H), 2.97 - 2.85 (m, 2H), 2.07 (s, 7H), 2.01 - 1.92 (m, 1H), 1.75 - 1.66 (m, 2H), 1.60 - 1.51 (m, 1H), 1.49 - 1.40 (m, 1H), 1.14 (d, J = 6.5 Hz, 3H).

### Example 6. Preparation of (S)-5-(((2-hydroxyethyl)(methyl)amino)methyl)-N-(3'-(5-(((1-hydroxypropan-2-yl)amino)methyl)-4-methoxypicolinamido)-2,2'-dimethyl-[1,1'-biphenyl]-3-yl)-4-methoxypicolinamide - Compound (R1_C441)

### Preparation of Intermediates:

### Intermediate (1.13b - PG): 5-({[(2S)-1-[(tert-butyldimethylsilyl)oxy]propan-2-yl]amino}methyl)-N-[3'-(5-{[(3-hydroxypropyl)(methyl)amino]methyl}-4-methoxypyridine-2-amido)-2,2'-dimethyl-[1,1'-biphenyl]-3-yl]-4-methoxypyridine-2-carboxamide

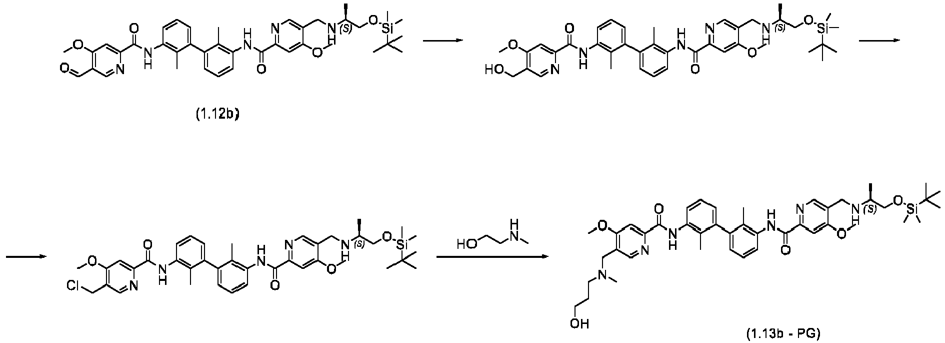

4M solution of lithium borohydride in tetrahydrofuran (0.42 mL, 1.7 mmol, 6.0 eq) was added to a solution of intermediate **(12b)** (0.2 g, 0.28 mmol, 1 eq) in tetrahydrofuran and methanol (1:1). The reaction mixture was allowed to stir for one hour at room temperature. Afterwards, it was quenched with several mL of water and concentrated in vacuo, the residue was taken up in dichloromethane, washed with water, the organic layer dried and concentrated to give desired product (0.17 g, 84% yield) which was analysed and used further.

¹H NMR (600 MHz, DMSO) δ 10.38 (d, J = 9.8 Hz, 2H), 8.52 (d, J = 10.4 Hz, 2H), 7.96 - 7.83 (m, 2H), 7.74 (d, J = 5.5 Hz, 2H), 7.33 (t, J = 7.8 Hz, 2H), 6.99 (dt, J = 7.6, 1.5 Hz, 2H), 5.33 (t, J = 5.6 Hz, 1H), 4.59 (dd, J = 5.5, 0.9 Hz, 2H), 3.98 (d, J = 2.7 Hz, 6H), 3.89 - 3.70 (m, 2H), 3.46 - 3.37 (m, 2H), 2.02 (s, 6H), 1.35 (s, 1H), 0.96 (d, J = 6.3 Hz, 3H), 0.84 (s, 10H), 0.00 (d, J = 7.4 Hz, 6H).

LC/MS: [M-H]⁺ = 714.46, 100% purity

Alcohol derivative intermediate (0.17 g, 0.24 mmol, 1 eq) was dissolved in dichloromethane and cooled to 0°C. Then triethylamine (0.099 mL, 0.7 mmol, 3 eq) and methanesulfonic chloride (0.027 mL, 0.36 mmol, 1.5 eq) was added and reaction was stirred for 30 min at 0°C. Then water was added and reaction was extracted with dichloromethane, washed with brine, dried over sodium sulphate and evaporated to give desired compound (18 mg, 35% yield) as yellowish foam. The crude material was used further (0.18 g, 0.25 mmol, 1 eq), 2-methylamino)ethanol (0.04 mL, 0.49 mmol, 2 eq), potassium carbonate (102 mg, 0.74 mmol, 3 eq) and potassium iodide (41 mg, 0.25 mmol, 1 eq) were mixed in acetonitrile and stirred at 50°C over the night. Afterwards, the solvent was concentrated. The residue was extracted with dichloromethane and water. Organic layer was dried over magnesium sulphate and concentrated. The crude material was purified by means of column chromatography with dichloromethane and ethyl acetate as an eluent. It allowed to obtain intermediate **(1.13b** - **PG)** (47 mg, 25% yield).

¹H NMR (600 MHz, DMSO) δ 10.37 (d, J = 7.0 Hz, 2H), 8.53 (d, J = 16.6 Hz, 2H), 7.91 (t, J = 8.0 Hz, 2H), 7.75 (d, J = 4.4 Hz, 2H), 7.33 (t, J = 7.8 Hz, 2H), 7.02 - 6.96 (m, 2H), 4.42 (t, J = 5.3 Hz, 1H), 4.00 - 3.96 (m, 6H), 3.89 - 3.71 (m, 2H), 3.60 (s, 2H), 3.52 (q, J = 5.9 Hz, 2H), 3.43 - 3.38 (m, 2H), 2.47 (t, J = 6.2 Hz, 2H), 2.21 (s, 3H), 2.02 (d, J = 2.1 Hz, 6H), 0.96 (d, J = 6.3 Hz, 3H), 0.84 (s, 9H), 0.00 (d, J = 7.4 Hz, 6H).

### Final compound (R1_C441): (S)-5-(((2-hydroxyethyl)(methyl)amino)methyl)-N-(3'-(5-(((1-hydroxypropan-2-yl)amino)methyl)-4-methoxypicolinamido)-2,2'-dimethyl-[1,1'-biphenyl]-3-yl)-4-methoxypicolinamide

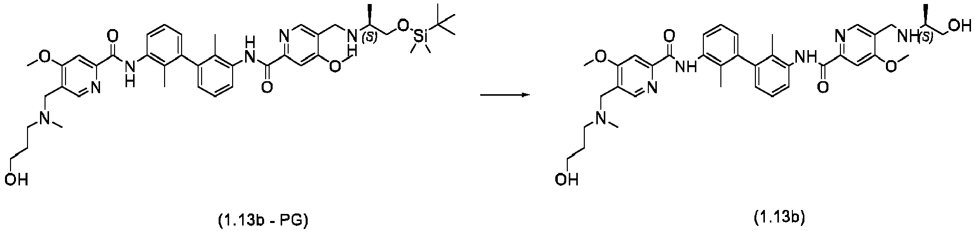

Intermediate **(1.13b** - **PG)** (41 mg, 0.05 mmol, 1 eq) was dissolved in dichloromethane. 6M solution of hydrochloric acid in isopropanol (0.086 mL, 0.51 mmol, 10 eq) was added. The reaction mixture was stirred at room temperature for four hours, when TLC indicated completion of reaction. It was extracted with 1M aqueous solution of sodium hydroxide, dried over magnesium sulphate and concentrated. The desired product was purified by means of preparative TLC with 30 % of methanol in dichloromethane as an eluent. Obtained material gave desired product **(1.13b, R1_C441)** (13 mg, 37% yield) as yellowish solid.

¹H NMR (600 MHz, MeOD) δ 8.50 (s, 1H), 8.45 (s, 1H), 7.94 (dt, J = 8.1, 1.5 Hz, 2H), 7.83 (d, J = 8.8 Hz, 2H), 7.31 (t, J = 7.8 Hz, 2H), 7.02 (dd, J = 7.6, 1.3 Hz, 2H), 4.04 (s, 3H), 4.01 (s, 3H), 3.93 (d, J = 13.8 Hz, 1H), 3.82 (d, J = 13.8 Hz, 1H), 3.69 (t, J = 6.0 Hz, 2H), 3.67 (s, 2H), 3.51 (dd, J = 10.9, 4.5 Hz, 1H), 3.38 - 3.34 (m, 1H), 2.77 - 2.67 (m, 1H), 2.60 (t, J = 6.0 Hz, 2H), 2.28 (s, 3H), 2.08 (d, J = 1.6 Hz, 6H), 1.06 (d, J = 6.4 Hz, 3H).

LC/MS: [M+H]⁺ = 657.44; 3.85 min, 100% purity

### Example 7. Preparation of 5-(((1,3-dihydroxypropan-2-yl)amino)methyl)-N-(3'-(5-((((1R,2R)-2-hydroxycyclopentyl)amino)methyl)-4-methoxypicolinamido)-2,2'-dimethyl-[1,1'-biphenyl]-3-yl)-4-methoxypicolinamide - Compound (R1_C445)

### Preparation of Intermediates:

### Intermediate (1.11b): 5-{[(1,3-dihydroxypropan-2-yl)amino]methyl}-N-[3'-(5-ethenyl-4-methoxypyridine-2-amido)-2,2'-dimethyl-[1,1'-biphenyl]-3-yl]-4-methoxypyridine-2-carboxamide

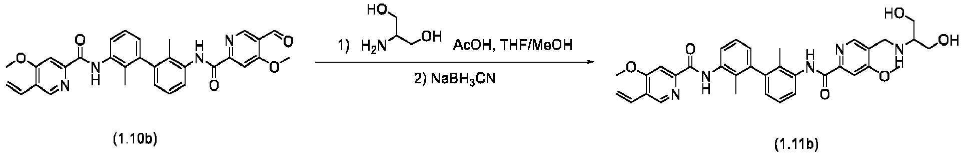

A mixture of intermediate **(1.10b)** (0.84 g, 1.57 mmol, 1 eq), 2-aminipropane-1,3-diol (0.713 g, 7.83 mmol, 5 eq.) and two drops of acetic acid in methanol and tetrahydrofuran (1:1) was stirred at 60°C for three hours. Afterwards, the reaction mixture was cooled down to 0°C and sodium cyanoborohydride (0.492 g, 7.83 mmol, 5 eq.) was added. Stirring was continued at room temperature over the night, when TLC indicated the completion of the reaction. It was quenched with water and the solvent was concentrated and the residue was extracted with dichloromethane and water. Organic layer was washed with brine and dried over magnesium sulphate and concentrated. The crude material was used further without any other purification as intermediate **(1.11b)** (0.84 g, 87% yield).

LC/MS: [M-H]⁺ = 612.31, 60% purity, 6.3 min

### Intermediate (1.11b - PG): N-[3'-(5-ethenyl-4-methoxypyridine-2-amido)-2,2'-dimethyl-[1,1'-biphenyl]-3-yl]4-methoxy-5-{[(2,2,3,3,9,9,10,10-octamethyl-4,8-dioxa-3,9-disilaundecan-6-yl)amino]methyl}pyridine-2-carboxamide

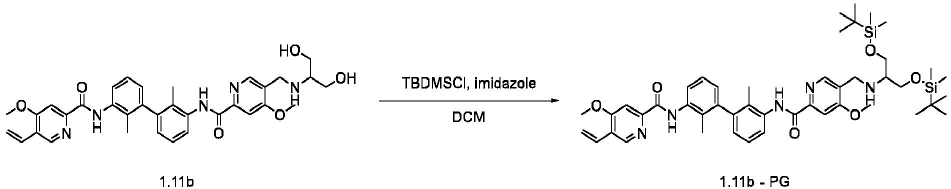

Compound **(1.11b)** (0.84 g, 1.37 mmol, 1 eq) was dissolved in dichloromethane. Imidazole (0.3 g, 4.4 mmol, 3.2 eq) and tert-butyldimethylchlorosilane (0.66 g, 4.4 mmol, 3.2 eq) were added. It was stirred at room temperature over the night. Afterwards, the mixture was extracted with dichloromethane and water. The combined organic layer was washed with brine, dried over magnesium sulphate and concentrated. The crude material was purified by means of flash column chromatography with ethyl acetate and hexane as an eluent to give compound **(1.11b-PG)** (0.76 g, yield 66%).

### Intermediate (1.12b): N-[3'-(5-ethenyl-4-methoxypyridine-2-amido)-2,2'-dimethyl-[1,1'-biphenyl]-3-yl]-4-methoxy-5-{[(2,2,3,3,9,9,10,10-octamethyl-4,8-dioxa-3,9-disilaundecan-6-yl)amino]methyl}pyridine-2-carboxamide

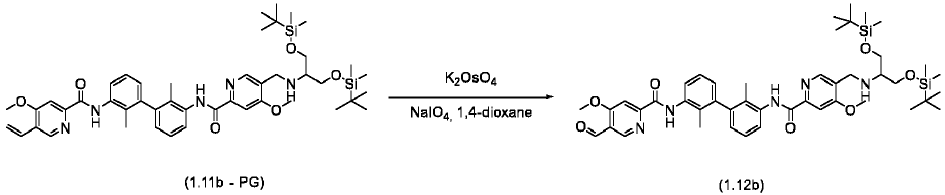

Compound **(1.11b - PG)** (0.76 g, 0.9 mmol, 1 eq) was dissolved in 1,4-dioxane and water (5:1). Potassium osmate dihydrate (17 mg, 0.04 mmol, 0.05 eq) and sodium periodate (0.58 g, 2.7 mmol, 3 eq) were added. Argon was bubbled through the reaction mixture. It was stirred at room temperature for 3 hours. Afterwards, the mixture was concentrated and extracted with dichloromethane and water. The combined organic layer was washed with brine, dried over magnesium sulphate and concentrated. The crude material was purified by means of flash column chromatography with ethyl acetate and hexane as an eluent to give compound **(1.12b)** (0.25 g, 33%).

LC/MS: [M-H]⁺ = 842.55, 93% purity, 9.2 min

¹H NMR (600 MHz, DMSO) δ 10.49 (s, 1H), 10.39 (s, 1H), 10.37 (s, 1H), 8.84 (s, 1H), 8.53 (s, 1H), 7.95 (s, 1H), 7.92 (d, J = 8.1 Hz, 1H), 7.78 (d, J = 8.1 Hz, 1H), 7.74 (s, 1H), 7.34 (td, J = 7.8, 3.5 Hz, 2H), 7.03 (d, J = 7.6 Hz, 1H), 6.99 (d, J = 7.6 Hz, 1H), 4.13 (s, 3H), 3.97 (s, 3H), 3.85 (s, 2H), 3.54 (d, J = 5.6 Hz, 4H), 2.25 (s, 1H), 2.25 - 2.20 (m, 1H), 2.01 (d, J = 5.3 Hz, 6H), 0.84 (s, 18H), 0.01 (d, J = 3.2 Hz, 12H).

### Intermediate (1.13b - PG): N-{3'-[5-({[(1S,2S)-2-hydroxycyclopentyl]amino}methyl)-4-methoxypyridine-2-amido]-2,2'-dimethyl-[1,1'-biphenyl]-3-yl} -4-methoxy-5-{[(2,2,3,3,9,9,10,10-octamethyl-4,8-dioxa-3,9-disilaundecan-6-yl)amino]methyl}pyridine-2-carboxamide

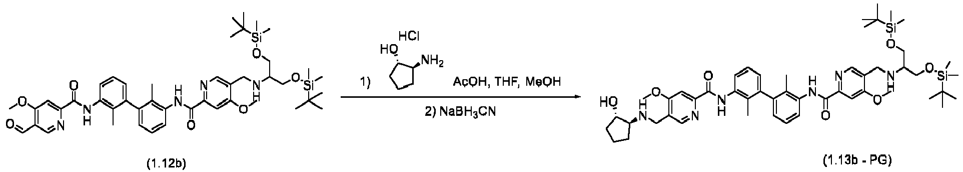

Compound **(1.12b)** (0.13 g, 0.15 mmol, 1 eq), (1R,2R)-2-aminocyclopentanol hydrochloride (0.1 g, 0.7 mmol, 4.5 eq.), previously mixed with triethylamine (0.1 mL, 0.7 mmol, 4.5 eq) and two drops of acetic acid were stirred in MeOH (4 mL) and THF (4 mL) at room temperature over the night. Afterwards, the reaction mixture was cooled down to 0°C and sodium cyanoborohydride (44 mg, 0.7 mmol, 4.5 eq.) was added. Stirring was continued at room temperature for four hour, when TLC indicated the completion of the reaction. It was quenched with water and the solvent was concentrated. The residue was extracted with dichloromethane and water. Organic layer was washed with brine and dried over magnesium sulphate and concentrated. The crude material was purified by means of column chromatography with dichloromethane and ethyl acetate as an eluent, followed by 60% of methanol in ethyl acetate. It allowed to obtain compound **(1.13b** - **PG)** (79 mg, 55% yield).

¹H NMR (600 MHz, DMSO) δ 10.37 (s, 2H), 8.53 (d, J = 7.4 Hz, 2H), 7.92 (d, J = 8.0 Hz, 2H), 7.74 (d, J = 1.3 Hz, 2H), 7.33 (t, J = 7.8 Hz, 2H), 6.99 (d, J = 7.3 Hz, 2H), 4.57 - 4.49 (m, 1H), 3.98 (d, J = 10.5 Hz, 6H), 3.85 (s, 2H), 3.76 (dd, J = 14.8, 5.0 Hz, 3H), 3.54 (d, J = 5.6 Hz, 4H), 2.72 (q, J = 6.0 Hz, 1H), 2.02 (d, J = 3.5 Hz, 6H), 1.90 - 1.75 (m, 2H), 1.65 - 1.51 (m, 2H), 1.43 - 1.25 (m, 2H), 1.23 (s, 1H), 0.84 (s, 18H), 0.01 (d, J = 3.1 Hz, 12H).

### Final compound (R1_C445): Synthesis of 5-(((1,3-dihydroxypropan-2-yl)amino)methyl)-N-(3'-(5-((((1R,2R)-2-hydroxycyclopentyl)amino)methyl)-4-methoxypicolinamido)-2,2'-dimethyl-[1,1'-biphenyl]-3-yl)-4-methoxypicolinamide

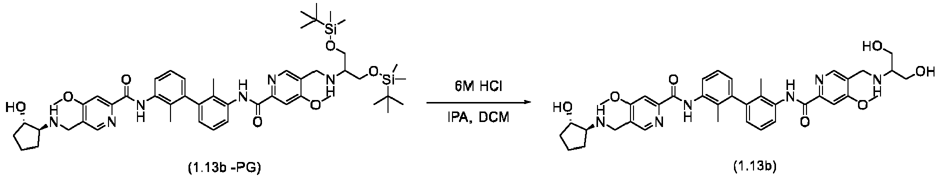

Compound **(1.13b** - **PG)** (79 mg, 0.09 mmol, 1 eq) was dissolved in dichloromethane. 6M solution of hydrochloric acid in isopropanol (0.213 mL, 1.3 mmol, 15 eq) was added. The reaction mixture was stirred at room temperature for two hours, when TLC indicated completion of reaction. It was extracted with 1M aqueous solution of sodium hydroxide, dried over magnesium sulphate and concentrated. The desired product was purified by means of preparative TLC with 30 % of methanol in dichloromethane as an eluent. Obtained material gave compound **(1.13b, R1_C445)** (13 mg, 22% yield).

LC/MS: [M+H]⁺ = 697.38; 3.9 min, 100% purity

¹H NMR (600 MHz, MeOD) δ 8.47 (d, J = 10.1 Hz, 2H), 7.95 (d, J = 9.4 Hz, 2H), 7.84 (s, 2H), 7.32 (t, J = 7.8 Hz, 2H), 7.03 (d, J = 7.6 Hz, 2H), 4.04 (d, J = 1.7 Hz, 6H), 3.94 (s, 2H), 3.93 (d, J = 5.9 Hz, 1H), 3.91 - 3.85 (m, 2H), 3.64 (dd, J = 11.1, 5.3 Hz, 2H), 3.55 (dd, J = 11.1, 6.1 Hz, 2H), 2.89 - 2.82 (m, 1H), 2.75 - 2.66 (m, 1H), 2.08 (s, 6H), 2.08 - 2.00 (m, 1H), 2.01 - 1.91 (m, 1H), 1.77 - 1.65 (m, 2H), 1.57 - 1.50 (m, 1H), 1.47 - 1.38 (m, 1H).

### Example 8. Preparation of (S)-5-(((1,3-dihydroxypropan-2-yl)amino)methyl)-N-(3'-(5-(((1-hydroxypropan-2-yl)amino)methyl)-4-methoxypicolinamido)-2,2'-dimethyl-[1,1'-biphenyl]-3-yl)-4-methoxypicolinamide - Compound (R1_C446)

### Preparation of Intermediates:

### Intermediate (1.13b - PG): N-{3'-[5-({[(2S)-1-hydroxypropan-2-yl]amino}methyl)-4-methoxypyridine-2-amido]-2,2'-dimethyl-[1,1'-biphenyl]-3-yl} -4-methoxy-5-{[(2,2,3,3,9,9,10,10-octamethyl-4,8-dioxa-3,9-disilaundecan-6-yl)amino]methyl}pyridine-2-carboxamide

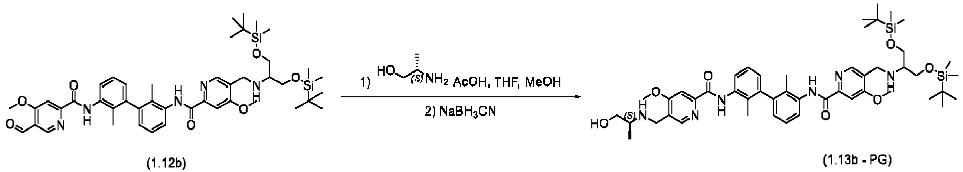

Compound **(1.12b)** (0.12 g, 0.14 mmol, 1 eq), (S)-2-aminopropan-1-ol (55 µL, 0.7 mmol, 5 eq.) and two drops of acetic acid were stirred in MeOH (4 mL) and THF (4 mL) at room temperature over the night. Afterwards, the reaction mixture was cooled down to 0°C and sodium triacetoxyborohydride (151 mg, 0.7 mmol, 5 eq.) was added. Stirring was continued at room temperature for one hour, when TLC indicated the completion of the reaction. It was quenched with water and the solvent was concentrated. The residue was extracted with dichloromethane and water. Organic layer was washed with brine and dried over magnesium sulphate and concentrated. The crude material was purified by means of column chromatography with dichloromethane and ethyl acetate as an eluent, followed by 60% of methanol in ethyl acetate. It allowed to obtain compound **(1.13b** - **PG)** (51 mg, 40% yield).

¹H NMR (600 MHz, DMSO) δ 10.46 - 10.34 (m, 2H), 8.66 - 8.49 (m, 2H), 7.99 - 7.71 (m, 4H), 7.34 (td, J = 7.8, 3.2 Hz, 2H), 7.00 (dd, J = 15.4, 7.6 Hz, 2H), 4.60 - 4.54 (m, 1H), 4.04 (s, 3H), 3.97 (s, 3H), 3.85 (s, 2H), 3.61 - 3.40 (m, 7H), 3.24 (d, J = 4.9 Hz, 1H), 3.17 (d, J = 5.2 Hz, 1H), 2.01 (d, J = 2.6 Hz, 6H), 1.23 (s, 3H), 0.84 (s, 18H), 0.02 (s, 12H).

### Final compound (R1_C446): Synthesis of (S)-5-(((1,3-dihydroxypropan-2-yl)amino)methyl)-N-(3'-(5-(((1-hydroxypropan-2-yl)amino)methyl)-4-methoxypicolinamido)-2,2'-dimethyl-[1,1'-biphenyl]-3-yl)-4-methoxypicolinamide

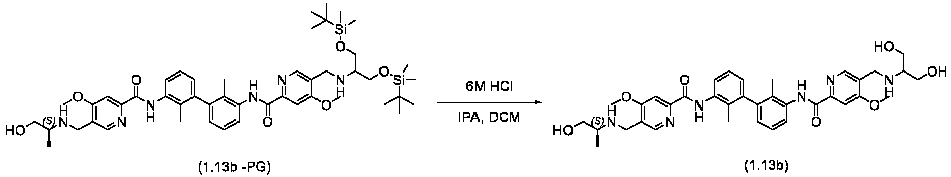

Compound **(1.13b** - **PG)** (51 mg, 0.06 mmol, 1 eq) was dissolved in dichloromethane. 6M solution of hydrochloric acid in isopropanol (0.141 mL, 0.85 mmol, 15 eq) was added. The reaction mixture was stirred at room temperature for two hours, when TLC indicated completion of reaction. It was extracted with 1M aqueous solution of sodium hydroxide, dried over magnesium sulphate and concentrated. Some precipitate crystallised from aqueous phase. White solid was filtered of and analysed as a compound **(1.13b, R1_C446)** (0.5 mg).

LC/MS: [M+H]⁺ = 673.46, 3.85 min

¹H NMR (600 MHz, MeOD) δ 8.48 (d, J = 11.7 Hz, 2H), 7.94 (d, J = 8.0 Hz, 2H), 7.86 (d, J = 2.7 Hz, 2H), 7.33 (t, J = 7.8 Hz, 2H), 7.05 (dd, J = 7.7, 1.3 Hz, 2H), 4.05 (d, 6H), 3.98 - 3.94 (m, 3H), 3.84 (d, J = 13.7 Hz, 1H), 3.63 (dd, J = 11.1, 5.4 Hz, 2H), 3.56 - 3.49 (m, 3H), 3.34 (s, 1H), 2.77 - 2.67 (m, 2H), 2.09 (s, 6H), 1.07 (d, J = 6.3 Hz, 3H).

### Example 9. Preparation of (S)-5-(((1-hydroxypropan-2-yl)amino)methyl)-N-(3'-(5-(((3-hydroxypropyl)amino)methyl)-4-methoxypicolinamido)-2,2'-dimethyl-[1,1'-biphenyl]-3-yl)-4-methoxypicolinamide - Compound (R1_C453)

### Preparation of Intermediates:

### Intermediate (1.11b): N-[3'-(5-ethenyl-4-methoxypyridine-2-amido)-2,2'-dimethyl-[1,1'-biphenyl]-3-yl]-5-{[(3-hydroxypropyl)amino]methyl}-4-methoxypyridine-2-carboxamide

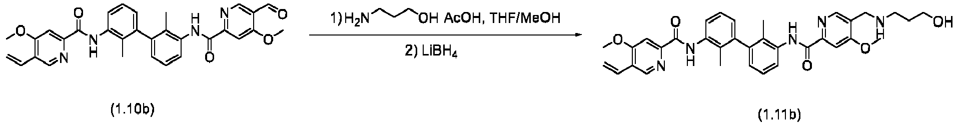

A mixture of intermediate **(1.10b)** (0.91 g, 1.7 mmol, 1 eq), 3-Amino-1-propanol (0.713 mL, 9.3 mmol, 5.5 eq.) and two drops of acetic acid in methanol and tetrahydrofuran (1:1) was stirred at 70°C for three hours. Afterwards, the reaction mixture was cooled down to 0°C and 4M lithium borohydride in tetrahydrofuran (2.33 mL, 9.33 mmol, 5.5 eq.) was added. Stirring was continued at room temperature for 10 min, when TLC indicated the completion of the reaction. It was quenched with water and the solvent was concentrated and the residue was extracted with dichloromethane and water. Organic layer was washed with brine and dried over magnesium sulphate and concentrated. The crude material was used further without any other purification as intermediate **(1.11b)** (1.0 g, 99% yield).

LC/MS: [M-H]⁺ = 596.36, 100%, 6.48 min

### Intermediate (1.11b - PG): 5-[({3-[(tert-butyldimethylsilyl)oxy]propyl}amino)methyl]-N-[3'-(5-ethenyl-4-methoxypyridine-2-amido)-2,2'-dimethyl-[1,1'-biphenyl]-3-yl]-4-methoxypyridine-2-carboxamide

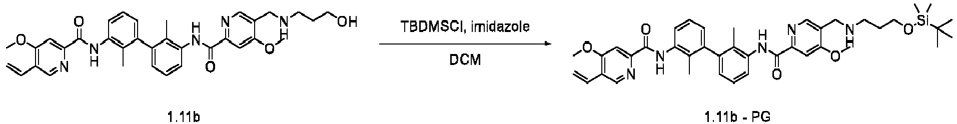

Compound **(1.11b)** (1.0 g, 1.68 mmol, 1 eq) was dissolved in dichloromethane. Imidazole (0.23 g, 3.36 mmol, 2 eq) and tert-butyldimethylchlorosilane (0.3 g, 2.0 mmol, 1.2 eq) were added. It was stirred at room temperature over the night. Afterwards, the mixture was extracted with dichloromethane and water. The combined organic layer was washed with brine, dried over magnesium sulphate and concentrated. The crude material was purified by means of flash column chromatography with ethyl acetate and hexane as an eluent to give compound **(1.11b** - **PG)** (0.7 g, yield 58%).

LC/MS: [M-H]⁺ = 710.41, 82%, 8.55 min

### Intermediate (1.12b): 5-[({3-[(tert-butyldimethylsilyl)oxy]propyl}amino)methyl]-N-[3'-(5-formyl-4-methoxypyridine-2-amido)-2,2'-dimethyl-[1,1'-biphenyl]-3-yl]-4-methoxypyridine-2-carboxamide

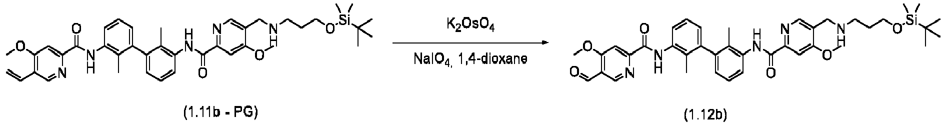

Compound **(1.11b-PG)** (0.7 g, 1.12mmol, 1 eq) was dissolved in 1,4-dioxane and water (5:1). Potassium osmate dihydrate (21 mg, 0.06 mmol, 0.05 eq) and sodium periodate (0.48 g, 2.24 mmol, 2 eq) were added. Argon was bubbled through the reaction mixture. It was stirred at room temperature for 3 hours. Afterwards, the mixture was concentrated and extracted with dichloromethane and water. The combined organic layer was washed with brine, dried over magnesium sulphate and concentrated. The crude material was purified by means of flash column chromatography with ethyl acetate and hexane as an eluent to give compound **(1.12b)** (0.37 g, 53%).

¹H NMR (600 MHz, DMSO) δ 10.48 (d, J = 13.4 Hz, 1H), 10.37 (d, J = 12.0 Hz, 2H), 8.83 (d, J = 9.2 Hz, 1H), 8.50 (s, 1H), 7.97 - 7.88 (m, 2H), 7.82 - 7.66 (m, 2H), 7.38 - 7.28 (m, 2H), 7.06 - 6.96 (m, 2H), 4.12 (d, J = 4.6 Hz, 3H), 3.98 (s, 3H), 3.63 (t, J = 6.2 Hz, 2H), 2.01 (d, J = 7.1 Hz, 6H), 1.97 (d, J = 2.3 Hz, 2H), 1.67 - 1.55 (m, 2H), 1.23 (s, 3H), 0.87 - 0.80 (m, 9H), - 0.00 (s, 6H).

### Intermediate (1.13b - PG): N-(3'-{5-[({3-[(tert-butyldimethylsilyl)oxy]propyl} amino)methyl]-4-methoxypyridine-2-amido}-2,2'-dimethyl-[1,1-biphenyl]-3-yl)-5-({[(2S)-1-hydroxypropan-2-yl]amino}methyl)-4-methoxypyridine-2-carboxamide

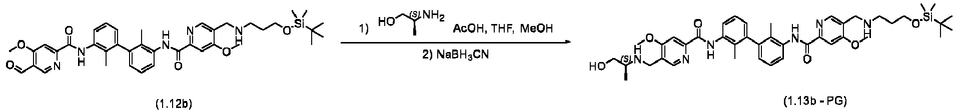

Compound **(1.12b)** (0.1 g, 0.14 mmol, 1 eq), (S)-2-aminopropan-1-ol (0.087 mL, 1.12 mmol, 8 eq.) and two drops of acetic acid were stirred in MeOH (4 mL) and THF (4 mL) at room temperature for 1h. Afterwards, the reaction mixture was cooled down to 0°C and sodium cyanoborohydride (71 mg, 1.12 mmol, 8 eq.) was added. Stirring was continued at room temperature for one hour, when TLC indicated the completion of the reaction. It was quenched with water and the solvent was concentrated. The residue was extracted with dichloromethane and water. Organic layer was washed with brine and dried over magnesium sulphate and concentrated. The crude material was purified by means of column chromatography with dichloromethane and ethyl acetate as an eluent, followed by 60% of methanol in ethyl acetate. It allowed to obtain compound **(1.13b** - **PG)** (53 mg, 40% yield).

¹H NMR (600 MHz, MeOD) δ 8.43 (d, J = 9.0 Hz, 2H), 7.97 (m, 2H), 7.82 (d, J = 3.8 Hz, 2H), 7.30 (t, J = 7.8 Hz, 2H), 7.01 (dd, J = 7.5, 1.3 Hz, 2H), 4.02 (d, J = 6.4 Hz, 6H), 3.97 - 3.88 (m, 1H), 3.86 - 3.80 (m, 3H), 3.73 - 3.65 (m, 2H), 3.59 - 3.50 (m, 1H), 3.39 - 3.29 (m, 3H), 2.78 - 2.66 (m, 3H), 2.07 (s, 6H), 1.79 - 1.68 (m, 2H), 1.07 (d, J = 6.4 Hz, 3H), 0.85 (s, 9H), 0.03 (s, 6H).

### Final compound (R1_C453): Synthesis of (S)-5-(((1-hydroxypropan-2-yl)amino)methyl)-N-(3'-(5-(((3-hydroxypropyl)amino)methyl)-4-methoxypicolinamido)-2,2'-dimethyl-[1,1'-biphenyl]-3-yl)-4-methoxypicolinamide

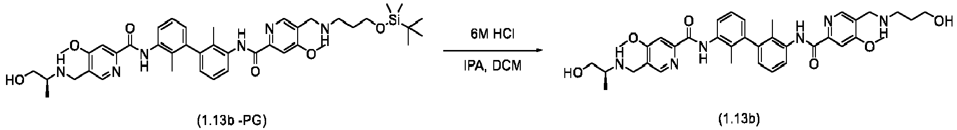

Compound **(1.13b** - **PG)** (53 mg, 0.07 mmol, 1 eq) was dissolved in dichloromethane. 6M solution of hydrochloric acid in isopropanol (0.17 mL, 1.0 mmol, 15 eq) was added. The reaction mixture was stirred at room temperature for two hours, when TLC indicated completion of reaction. It was concentrated. The desired product was purified by means of preparative TLC with 35 % of methanol in dichloromethane as an eluent. Obtained material gave compound **(1.13b, R1_C453)** (17 mg, 33% yield).

LC/MS: [M-H]⁻ = 655.24, 100% purity, 4.0 min

¹H NMR (600 MHz, MeOD) δ 8.52 (d, J = 13.9 Hz, 2H), 7.94 (m, 2H), 7.88 (d, J = 11.3 Hz, 2H), 7.32 (t, J = 7.8 Hz, 2H), 7.03 (dt, J = 7.6, 1.6 Hz, 2H), 4.12 (s, 2H), 4.08 - 3.97 (m, 7H), 3.69 (t, J = 5.9 Hz, 2H), 3.64 (dd, J = 11.3, 4.3 Hz, 1H), 3.46 (dd, J = 11.3, 7.3 Hz, 1H), 3.35 (s, 1H), 3.01 (t, J = 7.0 Hz, 2H), 2.96 (pd, J = 6.6, 4.4 Hz, 1H), 2.07 (s, 6H), 1.91 - 1.82 (m, 2H), 1.17 (d, J = 6.6 Hz, 3H).

### Example 10. Preparation of 5-(((2-hydroxyethyl)amino)methyl)-N-(3'-(5-(((3-hydroxypropyl)amino)methyl)-4-methoxypicolinamido)-2,2'-dimethyl-[1,1'-biphenyl]-3-yl)-4-methoxypicolinamide - Compound (R1_C454)

### Preparation of Intermediates:

### Intermediate (1.13b - PG): N-(3'-{5-[({3-[(tert-butyldimethylsilyl)oxy]propyl}amino)methyl]-4-methoxypyridine-2-amido}-2,2'-dimethyl-[1,1'-biphenyl]-3-yl)-5-{[(2-hydroxyethyl)amino]methyl} -4-methoxypyridine-2-carboxamide

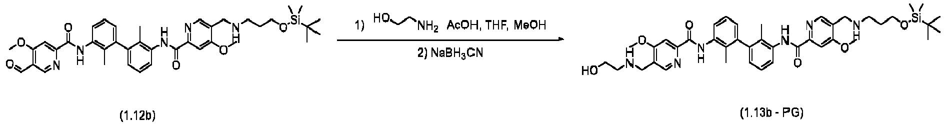

Compound **(1.12b)** (0.1 g, 0.14 mmol, 1 eq), ethanol amine (68 µL, 1.12 mmol, 8 eq.) and two drops of acetic acid were stirred in MeOH (4 mL) and THF (4 mL) at room temperature for one hour. Afterwards, the reaction mixture was cooled down to 0°C and sodium cyanoborohydride (71 mg, 1.12 mmol, 8 eq.) was added. Stirring was continued at room temperature for one hour, when TLC indicated the completion of the reaction. It was quenched with water and the solvent was concentrated. The residue was extracted with dichloromethane and water. Organic layer was washed with brine and dried over magnesium sulphate and concentrated. The crude material was purified by means of column chromatography with dichloromethane and ethyl acetate as an eluent, followed by 60% of methanol in ethyl acetate. It allowed to obtain compound **(1.13b** - **PG)** (15 mg, 14% yield).

¹H NMR (600 MHz, MeOD) δ 8.45 (d, J = 3.2 Hz, 2H), 7.94 (m, 2H), 7.85 (d, J = 2.4 Hz, 2H), 7.32 (t, J = 7.8 Hz, 2H), 7.03 (dd, J = 7.6, 1.3 Hz, 2H), 4.04 (d, J = 3.2 Hz, 6H), 3.88 (d, J = 12.0 Hz, 4H), 3.73 - 3.63 (m, 4H), 2.78 - 2.70 (m, 4H), 2.08 (s, 6H), 1.79 - 1.71 (m, 2H), 0.85 (s, 9H), 0.03 (s, 6H).

### Final compound (R1_C454): Synthesis of 5-(((2-hydroxyethyl)amino)methyl)-N-(3'-(5-(((3-hydroxypropyl)amino)methyl)-4-methoxypicolinamido)-2,2'-dimethyl- [1,1'-biphenyl] -3-yl)-4-methoxypicolinamide

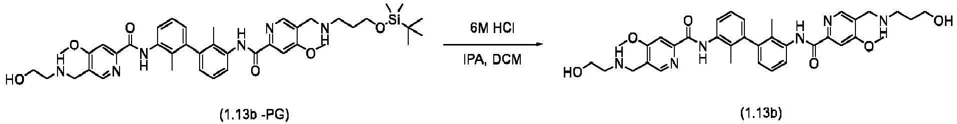

Compound **(1.13b** - **PG)** (15 mg, 0.02 mmol, 1 eq) was dissolved in dichloromethane. 6M solution of hydrochloric acid in isopropanol (0.05 mL, 0.3 mmol, 15 eq) was added. The reaction mixture was stirred at room temperature for two hours, when TLC indicated completion of reaction. It was concentrated. The crude material was purified by means of preparative TLC with 30% of methanol in dichloromethane. It allowed to obtain compound **(1.13b, R1_C454)** (4 mg, 31% yield).

LC/MS: [M-H]⁻ = 641.09, 100% purity, 3.99 min

¹H NMR (600 MHz, MeOD) δ 8.58 (d, J = 12.1 Hz, 2H), 7.97 - 7.89 (m, 4H), 7.34 (t, J = 7.8 Hz, 2H), 7.06 (dt, J = 7.6, 1.5 Hz, 2H), 4.28 (s, 2H), 4.22 (s, 2H), 4.11 (d, J = 6.5 Hz, 6H), 3.80 (dd, J = 6.1, 4.5 Hz, 2H), 3.73 (t, J = 5.8 Hz, 2H), 3.17 (t, J = 7.0 Hz, 2H), 3.05 (t, J = 5.3 Hz, 2H), 2.09 (s, 6H), 1.98 - 1.88 (m, 2H).

### Biological activity

### Determination of the affinity of compounds by HTRF method

The biological activity of the compounds described in the previous section was tested by the Homogenous Time Resolved Fluorescence (HTRF) method. Determination was carried out with the use of putty from the Cisbio company (catalog number 64ICP01PEG) in the 20 µL format using the protocol from the manufacturer. Compounds in the concentration range allowing determination of biological activity from the inflection curve were added to the hPD-1 (50 nM) and hPD-L1 (5 nM) protein complex with antibodies and allowed to incubate for one hour at room temperature before measuring Forster resonant energy transfer (TR-FRET) on a Tecan Spark 20M instrument. The results were normalized to a positive control (complex without inhibitor) and negative control (no hPD-1), averaged from three independent measurements and fitted with a Hill curve.

Ranges of activity of the tested compounds: "very good" (+++) below 20 nM, "moderate" (++) - 20-50 nM and "low" (+) - 50 nM - 1 µM.

### Determination of the activity of compounds by Blockade Bioassay

The potency of blocking the PD-1/PD-L1 pathway was assessed using the PD-1/PD-L1 Blockade Bioassay (Promega, cat. J1252). This assay relies on two genetically engineered cell lines: PD-1 Effector cells (Jurkat T cells expressing human PD-1 and luciferase driven by NFAT response element) and PD-L1 aAPC/CHO-K1 cells (CHO-K1 cells expressing human PD-L1 and cell surface protein designed to activate cognate T-cell Receptors in an antigen-independent manner). When the two cell types are co-cultured, the PD-1/PD-L1 interaction inhibits TCR signaling and NFAT-RE-mediated luminescence. Addition of either an anti-PD-1 or anti-PD-L1 antibody that blocks the PD-1/PD-L1 interaction releases the inhibitory signal and leads to TCR activation and the NFAT-RE-mediated luminescence, which is then measured on a microplate reader. The assay was performed according to the manufacturer's manual. PD-L1 aAPC/CHO-K1 cells were plated in 96-well, white, flat bottom assay plates at 40×10⁴ cells in 100 µL of medium (Ham's F12, 10% FBS) and incubated overnight at 37°C, 5% CO₂. The next day, the medium was removed from the assay plate and serially diluted compounds as listed in table 1 were added at 40 µL per well in the assay buffer (RPMI1640 + 1% FBS + 0,5% DMSO). Next, PD-1 Effector Jurkat cells were resuspended in the assay buffer (RPMI 1640 + 1% FBS) at a concentration of 1,25 × 10⁶ cells/mL and added to the assay plate at 40 µL per well (total of 50 × 10⁴ cells). The cells were co-cultured for 6 h (37°C, 5% CO₂) and then removed from the incubator and equilibrated for 5 min at room temperature. The Bio-GloTM Reagent (Promega, cat. G7941) was prepared according to the manufacturer's instructions and added to each well at 80 µL per well. The results were baseline-corrected to non-treated controls, averaged from three replications and fitted with a 4-parameters variable slope.

The compounds selected for the HTRF and NFAT activity studies are shown in Table 1.

**Table 1. HTRF and NFAT Cellular Assay Results**

| ID_number, name and structure | HTRF | PD-1/PD-L1 Blockade Bioassay EC₅₀ (nM) |
|---|---|---|
| R1_C428 | +++ | 44.6 nM |
| 5-((((1S,2S)-2-hydroxycyclopentyl)amino)methyl)-N-(3'-(5-((((S)-1-hydroxypropan-2-yl)amino)methyl)-4-methoxypicolinamido)-2,2'-dimethyl-[1,1'-biphenyl]-3-yl)-4-methoxypicolinamide | | |
| 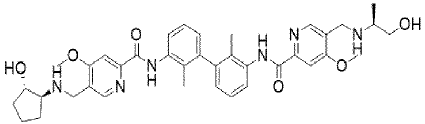 | | |
| R1_C441 | +++ | 176.0 nM |
| (S)-5-(((2-hydroxyethyl)(methyl)amino)methyl)-N-(3'-(5-(((1-hydroxypropan-2-yl)amino)methyl)-4-methoxypicolinamido)-2,2'-dimethyl-[1,1'-biphenyl]-3-yl)-4-methoxypicolinamide | | |
| 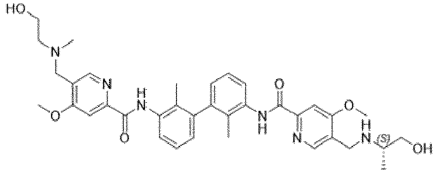 | | |
| R1_C445 | +++ | 150.0 nM |
| 5-(((1,3-dihydroxypropan-2-yl)amino)methyl)-N-(3'-(5-((((1R,2R)-2-hydroxycyclopentyl)amino)methyl)-4-methoxypicolinamido)-2,2'-dimethyl-[1,1'-biphenyl]-3-yl)-4-methoxypicolinamide | | |
| 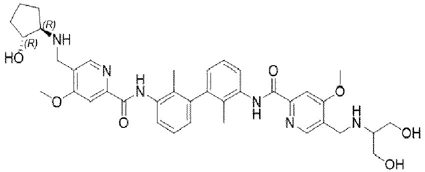 | | |
| R1_C446 | +++ | 205.0 nM |
| (S)-5-(((1,3-dihydroxypropan-2-yl)amino)methyl)-N-(3'-(5-(((1-hydroxypropan-2-yl)amino)methyl)-4-methoxypicolinamido)-2,2'-dimethyl-[1,1'-biphenyl]-3-yl)-4-methoxypicolinamide | | |
| 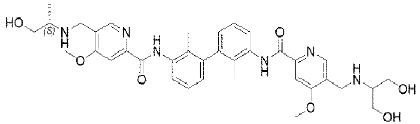 | | |
| R1_C450 | +++ | 206.0 nM |
| 5-(((2-hydroxyethyl)(methyl)amino)methyl)-N-(3' - (5-(((2-hydroxyethyl)(methyl)amino)methyl)picolinamido)-2,2'-dimethyl-[1,1'-biphenyl]-3-yl)-4-methoxypicolinamide | | |
| 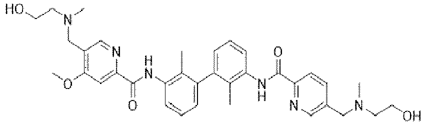 | | |
| R1_C451 | +++ | 68.0 nM |
| 5-((((1R,2R)-2-hydroxycyclopentyl)amino)methyl)-N-(3'-(5-(((2-hydroxyethyl)(methyl)amino)methyl)picolinamido)-2,2'-dimethyl-[1,1'-biphenyl]-3-yl)-4-methoxypicolinamide | | |
| 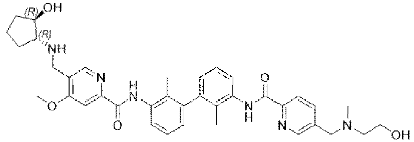 | | |
| R1_C451_1 | +++ | 233.7 nM |
| 5-((((1R,2R)-2-hydroxycyclopentyl)amino)methyl)-N-(3'-(5-(((2-hydroxyethyl)(methyl)amino)methyl)picolinamido)-2,2'-dimethyl-[1,1'-biphenyl]-3-yl)-4-methoxypicolinamide trihydrochloride | | |
| 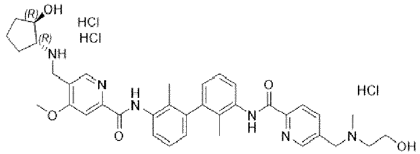 | | |
| R1_C453 | +++ | 143.4 nM |
| (S)-5-(((1-hydroxypropan-2-yl)amino)methyl)-N-(3'-(5-(((3-hydroxypropyl)amino)methyl)-4-methoxypicolinamido)-2,2'-dimethyl-[1,1'-biphenyl]-3-yl)-4-methoxypicolinamide | | |
| 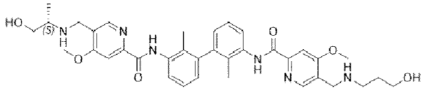 | | |
| R1_C454 | + | 480 nM |
| 5-(((2-hydroxyethyl)amino)methyl)-N-(3'-(5-(((3-hydroxypropyl)amino)methyl)-4-methoxypicolinamido)-2,2'-dimethyl-[1,1'-biphenyl] -3 -yl) -4-methoxypicolinamide | | |
| 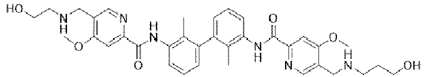 | | |
| R1_C458 | +++ | 159.3 nM |
| (S)-N-(3'-(5-(((2-hydroxyethyl)(methyl)amino)methyl)picolinamido)-2,2'-dimethyl-[1,1'-biphenyl]-3-yl)-5-(((1-hydroxypropan-2-yl)amino)methyl)-4-methoxypicolinamide | | |
| 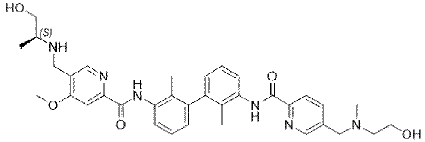 | | |

The activity of the compounds in dissociating the PD-1/PD-L1 complex has been confirmed by the HTRF tests and their cellular activity by the NFAT cellular assay. All compounds from table 1 exhibited EC50 in the NFAT cellular assay below 500 nM and are therefore considered potent to stimulate T-cells to clinically relevant anti-cancer activity.

Modulating the immune response by inhibiting the checkpoint receptors with antibodies is an effective method of fighting cancer known as immuno-oncology. The use of small molecule inhibitors instead of the known proteins is an advantageous change in cancer therapies due to the frequent and numerous adverse immunological effects caused by the use of antibodies.

In the light of the results of the tests described above, the compounds according to the invention constitute a new, attractive group of active inhibitors that bind directly to PD-L1, which was confirmed by the presented NMR tests, in which the compound dimerizes the protein, and the results of the HTRF tests performed.

### ABBREVIATIONS:

**AcOH** - acetic acid
**CHCl₃** - chloroform
**DCM** - dichloromethane
**CDCl₃** - deuterated chloroform
**DMF -** dimethylformamide
**DMSO** - dimethyl sulfoxide
**EDC** - N- (3-Dimethylaminopropyl) -N'-ethylcarbodiimide hydrochloride
**EtOAc** - ethyl acetate
**HATU** - Bis (dimethylamino) methylene 3-oxide hexafluorophosphate] -1H-1,2,3-triazolo [4,5-b] pyridinium 3-oxide
**Hex -** heksane
**HOBt** - 1-Hydroxybenzotriazole hydrate
**HTRF** - Homogenous Time Resolved Fluorescence
**K₂CO₃** - potassium carbonate
**LiBH₄** - sodium borohydride
**MeOH** - methanol
**MgSO₄** - magnesium sulphate (VI)
**MsCl** - Methanesulfonyl chloride
**NaBH₃CN** - sodium cyanoborohydride
**NaHCO₃** - sodium bicarbonate
**NMR** - Nuclear Magnetic Resonance Spectroscopy
**PD-1** - Programmed Death 1
**PD-L1 -** Programmed Death Ligand
**Pd(dppf)Cl₂** - [1,1'-bis (diphenylphosphino) ferrocene] dichloropalladium (II)
**pTLC** - preparative thin layer chromatography
**RT -** room temperature
**THF** - tetrahydrofuran;
**TLC** - thin layer chromatography
**TR-FRET** - Forster transfer of resonant energy
**TBDMSCl** - tert-butyldimethylsilyl chloride
**TMS** - tetramethylsilane

Literature:
1. Hellmann, M. D. et al. Nivolumab plus Ipilimumab in Advanced Non-Small-Cell Lung Cancer. N. Engl. J. Med. 381, 2020-2031 (2019).
2. Hellmann, M. D. et al. Nivolumab plus ipilimumab in lung cancer with a high tumor mutational burden. N. Engl. J. Med. (2018) doi:10.1056/NEJMoa1801946.
3. Wolchok, J. D. et al. Nivolumab plus Ipilimumab in advanced melanoma. N. Engl. J. Med. (2013) doi:10.1056/NEJMoa1302369.
4. Pardoll, D. M. The blockade of immune checkpoints in cancer immunotherapy. Nat. Rev. Cancer 12, 252-264 (2012).
5. Tumeh, P. C. et al. PD-1 blockade induces responses by inhibiting adaptive immune resistance. Nature 515, 568-71 (2014).
6. Adams, J. L., Smothers, J., Srinivasan, R. & Hoos, A. Big opportunities for small molecules in immuno-oncology. Nat. Rev. Drug Discov. 14, 603-622 (2015).

## Claims

1. Compound of general formula (I): 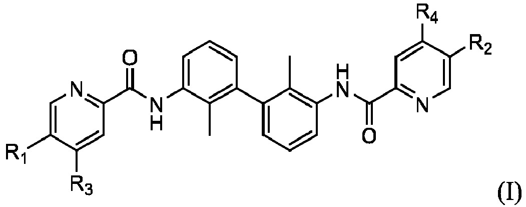 or a pharmaceutically acceptable salt or a stereoisomer thereof, wherein:
R₁ and R₂ are each independently selected from the group consisting of: 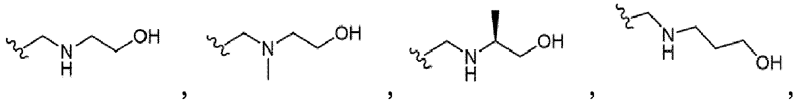 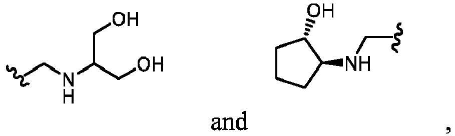 wherein R₂ is different from R₁,
R₃ and R₄ are each independently selected from -H and -OCH₃,
and wherein at least one of R₃ and R₄ is the -OCH₃ group.

2. The compound of claim 1 or pharmaceutically acceptable salt or stereoisomer thereof, wherein R₃ is -OCH₃.

3. The compound of claim 1 or pharmaceutically acceptable salt or stereoisomer thereof, wherein R₄ is -OCH₃.

4. The compound of claim 1 or 2 or 3, wherein the compound is selected from:
5-((((1S,2S)-2-hydroxycyclopentyl)amino)methyl)-N-(3'-(3-((((S)-1-hydroxypropan-2-yl)amino)methyl)-4-methoxypicolinamido)-2,2' -dimethyl-[1,1'-biphenyl]-3-yl)-4-methoxypicolinamide;
(S)-5-(((2-hydroxyethyl)(methyl)amino)methyl)-N-(3'-(5-(((1-hydroxypropan-2-yl)amino)methyl)-4-methoxypicolinamido)-2,2'-dimethyl-[1,1'-biphenyl]-3-yl)-4-methoxypicolinamide;
5-(((1,3-dihydroxypropan-2-yl)amino)methyl)-N-(3'-(5-((((1R,2R)-2-hydroxycyclopentyl)amino)methyl)-4-methoxypicolinamido)-2,2' -dimethyl-[1,1'-biphenyl]-3-yl)-4-methoxypicolinamide;
(S)-5-(((1,3-dihydroxypropan-2-yl)amino)methyl)-N-(3'-(5-(((1-hydroxypropan-2-yl)amino)methyl)-4-methoxypicolinamido)-2,2' -dimethyl-[1,1'-biphenyl]-3-yl)-4-methoxypicolinamide;
5-(((2-hydroxyethyl)(methyl)amino)methyl)-N-(3'-(5-(((2-hydroxyethyl)(methyl)amino)methyl)picolinamido)-2,2'-dimethyl-[1,1'-biphenyl]-3-yl)-4-methoxypicolinamide;
5-((((1R,2R)-2-hydroxycyclopentyl)amino)methyl)-N-(3'-(5-(((2-hydroxyethyl)(methyl)amino)methyl)picolinamido)-2,2'-dimethyl-[1,1'-biphenyl]-3-yl)-4-methoxypicolinamide;
5-((((1R,2R)-2-hydroxycyclopentyl)amino)methyl)-N-(3'-(5-(((2-hydroxyethyl)(methyl)amino)methyl)picolinamido)-2,2'-dimethyl-[1,1'-biphenyl]-3-yl)-4-methoxypicolinamide trihydrochloride;
(S)-5-(((1-hydroxypropan-2-yl)amino)methyl)-N-(3'-(5-(((3-hydroxypropyl)amino)methyl)-4-methoxypicolinamido)-2,2'-dimethyl-[1,1'-biphenyl]-3-yl)-4-methoxypicolinamide;
5-(((2-hydroxyethyl)amino)methyl)-N-(3' -(5-(((3-hydroxypropyl)amino)methyl)-4-methoxypicolinamido)-2,2'-dimethyl-[1,1'-biphenyl]-3-yl)-4-methoxypicolinamide;
(S)-N-(3'-(5-(((2-hydroxyethyl)(methyl)amino)methyl)picolinamido)-2,2'-dimethyl-[1,1'-biphenyl]-3-yl)-5-(((1-hydroxypropan-2-yl)amino)methyl)-4-methoxypicolinamide;
or a pharmaceutically acceptable salt or a stereoisomer thereof.

5. The compound of any one of claims 1-4 or a pharmaceutically acceptable salt or a stereoisomer thereof for use as a medicament, preferably in treatment and/or prophylaxis of malignant neoplastic diseases or disorders or in stimulating and/or increasing an immune response in a patient in response to an infectious disease, wherein the patient is a mammal, preferably a human.

6. The compound, pharmaceutically acceptable salt or a stereoisomer thereof for use according to claim 5, wherein the disease or disorder spreads through an organism through a PD-1/PD-L1 signaling pathway and the compound of general formula (I) is used as a modulator of the PD-1/PD-L1 signaling pathway.

7. The compound, pharmaceutically acceptable salt or a stereoisomer thereof for use according to claim 6, wherein the compound of general formula (I) binds to PD-L1 protein receptor and/or dimerizes PD-L1 protein receptor and/or inhibits PD-1/PD-L1 interaction.

8. Use of the compound of any one of claims 1-4, or a salt or stereoisomer thereof for inhibiting growth of tumor cells *in vitro,* wherein a tumor cell or tissue is contacted *in vitro* with a compound of general formula (I) or with a salt or stereoisomer thereof.
